(19) Europäisches Patentamt
European Patent Office
Office européen des brevets

(11) EP 4 458 792 B1

(12) EUROPEAN PATENT SPECIFICATION

(45) Date of publication and mention of the grant of the patent:
**29.04.2026 Bulletin 2026/18**

(21) Application number: **24173051.4**

(22) Date of filing: **29.04.2024**

(51) International Patent Classification (IPC):
***C07C 1/32*** *(2006.01)* ***C07C 29/36*** *(2006.01)*
***C07C 17/263*** *(2006.01)* ***C07C 17/16*** *(2006.01)*
***C07C 19/01*** *(2006.01)* ***C07C 9/22*** *(2006.01)*
***C07C 29/40*** *(2006.01)*

(52) Cooperative Patent Classification (CPC):
(C-Sets available)
**C07C 1/326; C07C 17/16; C07C 17/2632;**
**C07C 19/01; C07C 29/40** (Cont.)

(54) **1-HALO-2,6,14-TRIMETHYLOCTADECANE COMPOUND AND PROCESS FOR PREPARING 5,13,17-TRIMETHYLALKANE COMPOUND THEREFROM**

1-HALO-2,6,14-TRIMETHYLOCTADECAN-VERBINDUNG UND VERFAHREN ZUR HERSTELLUNG EINER 5,13,17-TRIMETHYLALKAN-VERBINDUNG DARAUS

COMPOSÉ 1-HALO-2,6,14-TRIMÉTHYLOCTADÉCANE ET PROCÉDÉ DE PRÉPARATION DE COMPOSÉ 5, 13,17-TRIMÉTHYLALCANE À PARTIR DE CELUI-CI

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **01.05.2023 JP 2023075366**

(43) Date of publication of application:
**06.11.2024 Bulletin 2024/45**

(73) Proprietor: **Shin-Etsu Chemical Co., Ltd.**
**Tokyo 100-0005 (JP)**

(72) Inventors:
- **MIYAKE, Yuki**
  **Niigata, 942-8601 (JP)**
- **SUGAWARA, Yuma**
  **Niigata, 942-8601 (JP)**
- **WATANABE, Takeru**
  **Niigata, 942-8601 (JP)**
- **KINSHO, Takeshi**
  **Niigata, 942-8601 (JP)**

(74) Representative: **De Vries & Metman**
**Overschiestraat 180**
**1062 XK Amsterdam (NL)**

(56) References cited:
- **BRANDT MIRIAM ET AL: "The scent of supercolonies: the discovery, synthesis and behavioural verification of ant colony recognition cues", BMC BIOLOGY, BIOMED CENTRAL, LONDON, GB, GB, vol. 7, no. 1, 28 October 2009 (2009-10-28), pages 71, XP021063661, ISSN: 1741-7007, DOI: 10.1186/ 1741-7007-7-71**
- **ELLEN VAN WILGENBURG ET AL: "Deciphering the Chemical Basis of Nestmate Recognition", JOURNAL OF CHEMICAL ECOLOGY, KLUWER ACADEMIC PUBLISHERS-PLENUM PUBLISHERS, NE, vol. 36, no. 7, 17 June 2010 (2010-06-17), pages 751 - 758, XP019822742, ISSN: 1573-1561**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 1/326, C07C 9/22;**
**C07C 17/16, C07C 19/01;**
**C07C 17/2632, C07C 19/01;**
**C07C 29/40, C07C 31/125**

## Description

TECHNICAL FIELD

The present invention relates to a 1-halo-2,6,14-trimethyloctadecane compound and a process for preparing a 5,13,17-trimethylalkane compound, the nest mate recognition pheromone of *Linepithema humile*, therefrom.

BACKGROUND ART

Ranked among the world's worst 100 invasive alien species, the Argentine ant (*Linepithema humile*) invades countries around the world, displaces native ants by forming supercolonies, and significantly impacts the ecosystem. Argentine ants also form symbiotic relationships that protect aphids and scale insects from natural enemies in return for the honeydew of these agricultural pests, often making biological control with natural enemies ineffective in regions of high Argentine ant density. Furthermore, Argentine ants are sanitary pests that invade households through slight gaps. Pesticide spraying has been used to control Argentine ants, but such traditional pesticide spraying is not very effective, and is undesirable from an environmental standpoint. Biological control methods that minimize the use of pesticides are therefore being studied, and promising pest control methods include the use of nest mate recognition pheromones (Non-Patent Literatures 1 and 2 below).

Known nest mate recognition pheromones of the Argentine ant (*Linepithema humile*) include 15-methylpentatriacontane; 17-methylpentatriacontane; 17-methylheptatriacontane; and 5,13,17-trimethylalkane compounds such as 5,13,17-trimethyltritriacontane, 5,13,17-trimethylpentatriacontane, and 5,13,17-trimethylheptatriacontane. Among these compounds, 5,13,17-trimethylalkane compounds such as 5,13,17-trimethyltritriacontane, 5,13,17-trimethylpentatriacontane, and 5,13,17-trimethylheptatriacontane; and 17-methylalkane compounds such as 17-methylpentatriacontane and 17-methylheptatriacontane are revealed to be especially active (Non-Patent Literatures 1, 2, and 3).

A process for synthesizing the aforesaid 5,13,17-trimethylalkane compound has been reported (Non-Patent Literature 2 below) in which a yield of 0.84 to 3.17% is obtained as in the following eleven steps. For example, two equivalents of methyllithium are reacted with 8-bromooctanoic acid to synthesize 9-bromo-2-nonanone. The carbonyl group of 9-bromo-2-nonanone thus obtained is acetalized with ethylene glycol in the presence of an acid catalyst, *p*-toluenesulfonic acid, to synthesize 2-(7-bromoheptyl)-2-methyl-1,3-dioxolane. Then, 2-(7-bromoheptyl)-2-methyl-1,3-dioxolane thus obtained is converted into [7-(2-methyl-1,3-dioxolan-2-yl)heptyl]magnesium bromide by reacting with magnesium in tetrahydrofuran, followed by subjecting [7-(2-methyl-1,3-dioxolan-2-yl)heptyl]magnesium bromide thus obtained to a nucleophilic addition reaction with 2-hexanone to synthesize α-butyl-α,2-dimethyl-1,3-dioxolan-2-octanol. α-Butyl-α,2-dimethyl-1,3-dioxolan-2-octanol thus obtained is then subjected to a dehydration reaction in benzene in the presence of an acid catalyst, *p*-toluenesulfonic acid, followed by eliminating acetal with water and acetone in the presence of an acid catalyst, *p*-toluenesulfonic acid, to synthesize 10-methyl-9-tetradecen-2-one. [3-(2-methyl-1,3-dioxolan-2-yl) propyl]magnesium chloride, which is synthesized separately, is then subjected to a nucleophilic addition reaction with 10-methyl-9-tetradecen-2-one to synthesize α,2-dimethyl-α-(8-methyl-7-dodecen-1-yl)-1,3-dioxolane-2-butanol. α,2-Dimethyl-α-(8-methyl-7-dodecen-1-yl)-1,3-dioxolane-2-butanol thus obtained is subjected to a dehydration reaction in benzene in the presence of an acid catalyst, *p*-toluenesulfonic acid, followed by eliminating acetal with water and acetone in the presence of an acid catalyst, *p*-toluenesulfonic acid, to synthesize 6,14-dimethyl-5,13-octadecadien-2-one. Separately, an alkyltriphenylphosphonium bromide compound is then subjected to a deprotonation reaction with n-butyllithium to prepare a triphenylphosphonium alkylide compound. The aforesaid triphenylphosphonium alkylide compound and the aforesaid 6,14-dimethyl-5,13-octadecadien-2-one are subjected to a Wittig reaction to synthesize a 5,13,17-trimethylalkatriene compound. Then, the 5,13,17-trimethylalkatriene compound thus obtained is subjected to a hydrogenation reaction in the presence of a palladium on carbon catalyst to obtain the aforesaid 5,13,17-trimethylalkane compound.

PRIOR ART

[Non-Patent Literatures]

[Non-Patent Literature 1] Neil D Tsutsui et al., BMC Biology, 2009, 7, 71.
[Non-Patent Literature 2] Neil D Tsutsui et al., J. Chem. Ecol., 2010, 36, 751-758.
[Non-Patent Literature 3] E. Sunamura et al., Insectes Sociaux, 2009, 56, 143-147.
[Non-Patent Literature 4] Dennis H. Burns et al., J. Am. Chem. Soc., 1997, 119, 2125-2133.

OBJECT OF THE INVENTION

However, the process for preparing the 5,13,17-trimethylalkane compound of Non-Patent Literature 2 is not industrially practical due to the use of benzene, which is extremely toxic to the human body, as a solvent. Industrialization also is made difficult by the use of n-butyllithium and palladium on carbon, which are ignitable. Moreover, the yields of the steps are generally low, with especially low yields of 22 to 45% for Grignard reagent addition and Wittig reactions.

The present invention has been made in view of the aforementioned circumstances, and aims to provide a novel compound that is a synthetic intermediate for efficiently preparing a 5,13,17-trimethylalkane compound. The present invention also aims to provide a process for preparing the aforesaid novel compound.

SUMMARY OF THE INVENTION

As a result of intensive research to overcome the aforesaid problems of the prior art, the present inventors found that a 1-halo-2,6,14-trimethyloctadecane compound according to the present invention is a novel compound, and that the 1-halo-2,6,14-trimethyloctadecane compound is a useful intermediate in the preparation of a 5,13,17-trimethylalkane compound. Furthermore, the present inventors found a preparation process in which the 5,13,17-trimethylalkane compound, the nest mate recognition pheromone of the Argentine ant (*Linepithema humile*), may be efficiently prepared with the 1-halo-2,6,14-trimethyloctadecane compound with fewer steps, and thus have completed the present invention. The preparation process also was found to be economic and industrially viable for preparing the 5,13,17-trimethylalkane compound.

According to a first aspect of the present invention, there is provided a 1-halo-2,6,14-trimethyloctadecane compound of the following general formula (1):

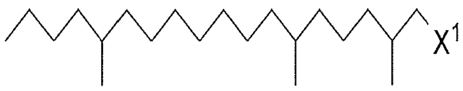
(1)

wherein $X^1$ represents a halogen atom.

According to a second aspect of the present invention, there is provided a process for preparing a 5,13,17-trimethylalkane compound of the following general formula (4):

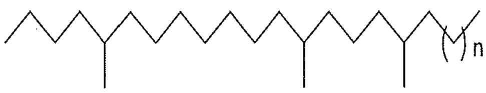
(4)

wherein n represents an integer of 14 to 18,
the process comprising
converting the aforesaid 1-halo-2,6,14-trimethyloctadecane compound (1) into a nucleophilic reagent, 2,6,14-trimethyloctadecyl, of the following general formula (2):

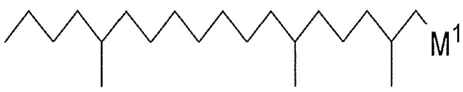
(2)

wherein $M^1$ represents Li or $MgZ^1$, and $Z^1$ represents a halogen atom or a 2,6,14-trimethyloctadecyl group,
and subsequently subjecting the nucleophilic reagent, 2,6,14-trimethyloctadecyl compound (2), to a coupling reaction with an electrophilic alkyl reagent of the following general formula (3):

$$CH_3(CH_2)_nX^2 \qquad (3)$$

wherein $X^2$ represents a halogen atom or a *p*-toluenesulfonyloxy group ($CH_3$-$C_6H_6$-$SO_2$-O(TsO) group), and *n* is as defined above,
to obtain the aforesaid 5,13,17-trimethylalkane compound (4).

According to a third aspect of the present invention, there is provided a process for preparing the aforesaid 1-halo-2,6,14-trimethyloctadecane compound of the following general formula (1):

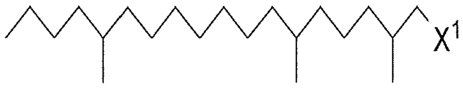 (1)

wherein $X^1$ represents a halogen atom,
the process comprising
converting a 1-halo-3,11-dimethylpentadecane compound of the following general formula (5):

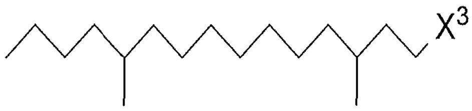 (5)

wherein $X^3$ represents a halogen atom,
into a nucleophilic reagent, 3,11-dimethylpentadecyl, of the following general formula (6):

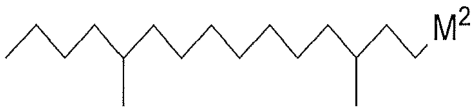 (6)

wherein $M^2$ represents Li or $MgZ^1$, and $Z^1$ represents a halogen atom or a 3,11-dimethylpentadecyl group,
and subsequently subjecting the nucleophilic reagent, 3,11-dimethylpentadecyl (6), to a coupling reaction with a 1,3-dihalo-2-methylpropane compound of the following general formula (7):

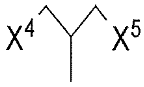 (7)

wherein $X^4$ and $X^5$ represent, independently of each other, a halogen atom,
to obtain the aforesaid 1-halo-2,6,14-trimethyloctadecane compound (1).

According to a fourth aspect of the present invention, there is provided the aforesaid process for preparing the 1-halo-2,6,14-trimethyloctadecane compound (1), the process further comprising
halogenating 3,11-dimethylpentadecanol of the following formula (8):

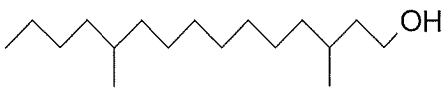 (8)

to obtain the aforesaid 1-halo-3,11-dimethylpentadecane compound (5).

According to a fifth aspect of the present invention, there is provided the aforesaid process for preparing the 1-halo-2,6,14-trimethyloctadecane compound (1), the process further comprising
converting a 2-halo-10-methyltetradecane compound of the following general formula (9):

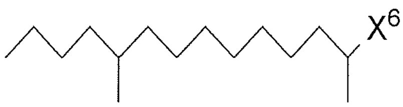 (9)

wherein $X^6$ represents a halogen atom,
into a nucleophilic reagent, 1,9-dimethyltridecyl, of the following general formula (10):

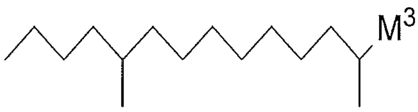 (10)

wherein $M^3$ represents Li or $MgZ^1$, and $Z^1$ represents a halogen atom or a 1,9-dimethyltridecyl group,

and subsequently subjecting the nucleophilic reagent, 1,9-dimethyltridecyl (10), to an addition reaction with ethylene oxide to obtain the aforesaid 3,11-dimethylpentadecanol (8).

According to a sixth aspect of the present invention, there is provided the aforesaid process for preparing the 1-halo-2,6,14-trimethyloctadecane compound (1), the process further comprising
halogenating 10-methyl-2-tetradecanol of the following formula (11):

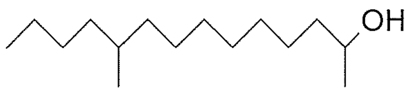
(11)

to obtain the aforesaid 2-halo-10-methyltetradecane compound (9).

According to the present invention, a 5,13,17-trimethylalkane compound can be prepared with fewer steps and with good yield, without using expensive starting materials. According to the present invention, a novel synthetic intermediate that is useful for preparing the 5,13,17-trimethylalkane compound also can be provided.

DETAILED DESCRIPTION OF THE INVENTION

A 1-halo-2,6,14-trimethyloctadecane compound, a novel compound, will be explained below in Section A.

A. 1-Halo-2,6,14-trimethyloctadecane compound of the following general formula (1)

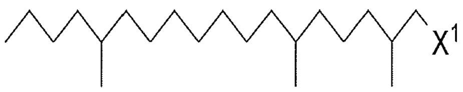
(1)

wherein $X^1$ represents a halogen atom.

Specific examples of the halogen atom $X^1$ include a chlorine atom, a bromine atom, and an iodine atom. The halogen atom $X^1$ is preferably a chlorine atom and a bromine atom, and more preferably a chlorine atom. By using said chlorine atom and bromine atom, a preferred availability may be ensured. By using said chlorine atom, a more preferred availability may be ensured.

Specific examples of the 1-halo-2,6,14-trimethyloctadecane compound (1) include 1-chloro-2,6,14-trimethyloctadecane, 1-bromo-2,6,14-trimethyloctadecane, and 1-iodo-2,6,14-trimethyloctadecane.

The 1-halo-2,6,14-trimethyloctadecane compound (1) can be prepared according to, for example, the following chemical reaction formula.

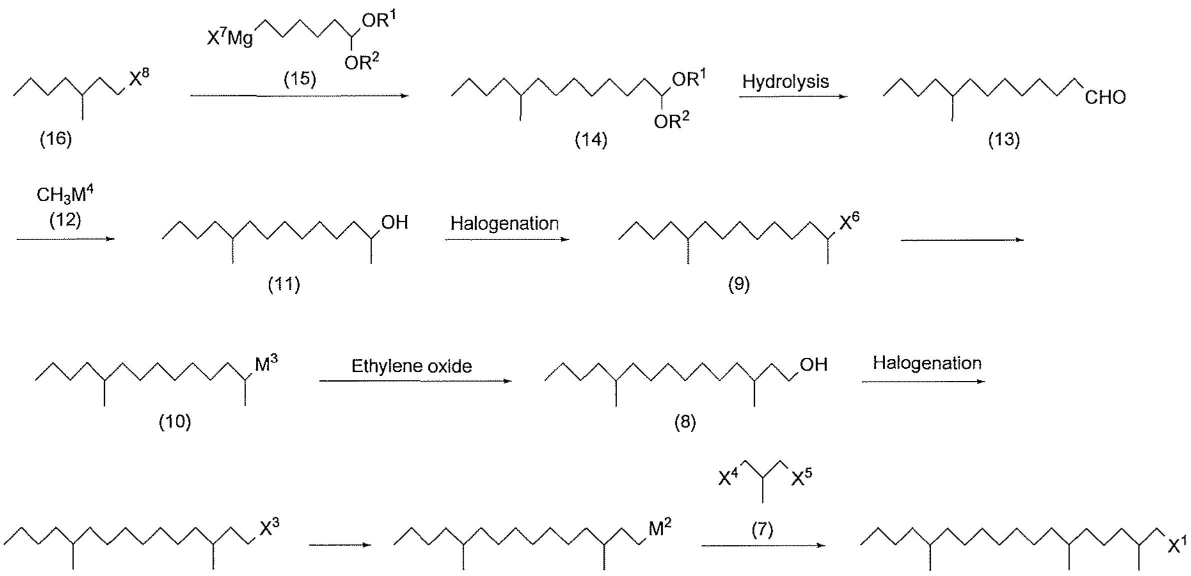

First, a 1-halo-3-methylheptane compound (16) is subjected to a coupling reaction with a 6,6-dialkoxyhexylmagnesium halide compound (15) to prepare a 1,1-dialkoxy-9-methyltridecane compound (14). The 1,1-dialkoxy-9-methyltridecane compound (14) thus obtained is then subjected to a hydrolysis reaction to prepare 9-methyltridecanal (13). 9-Methyltridecanal (13) thus obtained is then subjected to an addition reaction with a nucleophilic reagent, methyl (12), to prepare 10-methyl-2-tetradecanol (11). 10-Methyl-2-tetradecanol (11) thus obtained is halogenated to prepare a 2-halo-10-methyltetradecane compound (9). The 2-halo-10-methyltetradecane compound (9) thus obtained is then converted into a nucleophilic reagent, 1,9-dimethyltridecyl (10), after which the nucleophilic reagent, 1,9-dimethyltridecyl (10), thus obtained is reacted with ethylene oxide to prepare 3,11-dimethylpentadecanol (8). 3,11-Dimethylpentadecanol (8) thus obtained is then halogenated to prepare a 1-halo-3,11-dimethylpentadecane compound (5). The 1-halo-3,11-dimethylpentadecane compound (5) thus obtained is converted into a nucleophilic reagent, 3,11-dimethylpentadecyl (6), after which the nucleophilic reagent, 3,11-dimethylpentadecyl, thus obtained is subjected to a coupling reaction with a 1,3-dihalo-2-methylpropane compound to prepare the 1-halo-2,6,14-trimethyloctadecane compound (1).

The preparation of the 1,1-dialkoxy-9-methyltridecane compound (14), an intermediate useful in the preparation of the 1-halo-2,6,14-trimethyloctadecane compound (1), will be explained below in Section B.

B-1. 1,1-Dialkoxy-9-methyltridecane compound (14)

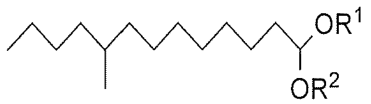

(14)

In the general formula (14), $R^1$ and $R^2$ represent, independently of each other, a monovalent hydrocarbon group having 1 to 15 carbon atoms, preferably 1 to 10 carbon atoms, and more preferably 1 to 5 carbon atoms; or $R^1$ and $R^2$ are bonded to each other and represent a divalent hydrocarbon group of $R^1$-$R^2$, having 2 to 10 carbon atoms, and preferably 2 to 5 carbon atoms.

Examples of the monovalent hydrocarbon group having 1 to 15 carbon atoms include linear saturated hydrocarbon groups such as a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group; branched saturated hydrocarbon groups such as an isopropyl group, a 2-methylpropyl group, and a 2-methylbutyl group; linear unsaturated hydrocarbon groups such as a 2-propenyl group; branched unsaturated hydrocarbon groups such as a 2-methyl-2-propenyl group; cyclic saturated hydrocarbon groups such as a cyclopropyl group; and isomers thereof. A part of the hydrogen atoms in the hydrocarbon groups may be substituted with a methyl group or an ethyl group.

The monovalent hydrocarbon group is preferably a methyl group, an ethyl group, an n-propyl group, and an n-butyl group. By using said methyl group, ethyl group, n-propyl group, and n-butyl group, a preferred handling may be ensured.

When $R^1$ and $R^2$ are bonded to each other to form a divalent hydrocarbon group, $R^1$-$R^2$, having 2 to 10 carbon atoms, examples include linear saturated hydrocarbon groups such as an ethylene group, a 1,3-propylene group, and a 1,4-butylene group; branched saturated hydrocarbon groups such as a 1,2-propylene group, a 2,2-dimethyl-1,3-propylene group, a 1,2-butylene group, a 1,3-butylene group, a 2,3-butylene group, and a 2,3-dimethyl-2,3-butylene group; linear unsaturated hydrocarbon groups such as a 1-vinylethylene group; branched unsaturated hydrocarbon groups such as a 2-methylene-1,3-propylene group; cyclic hydrocarbon groups such as a 1,2-cyclopropylene group and a 1,2-cyclobutylene group; and isomers thereof. A part of the hydrogen atoms in the hydrocarbon groups may be substituted with a methyl group or an ethyl group.

The divalent hydrocarbon group is preferably a lower hydrocarbon group, preferably having 2 to 4 carbon atoms with high reactivity and deprotection by-products that are easily removable by washing or concentration. By using said divalent hydrocarbon group, the reactivity of the deprotection, ease of purification, and/or availability may be ensured.

Particularly preferred examples of the divalent hydrocarbon group include an ethylene group, a 1,2-propylene group, a 1,3-propylene group, a 1,2-butylene group, a 1,3-butylene group, and a 2,3-dimethyl-2,3-butylene group in view of the aforesaid considerations.

Specific examples of the 1,1-dialkoxy-9-methyltridecane compound (14) include the following compounds: 1,1-dimethoxy-9-methyltridecane, 1,1-diethoxy-9-methyltridecane, 1,1-dipropyloxy-9-methyltridecane, 1,1-dibutyloxy-9-methyltridecane, 1,1-dipentyloxy-9-methyltridecane, 1,1-dihexyloxy-9-methyltridecane, 1,1-diheptyloxy-9-methyltridecane, 1,1-dioctyloxy-9-methyltridecane, 1,1-dinonyloxy-9-methyltridecane, 1,1-didecyloxy-9-methyltridecane, 2-(8-methyldodecyl)-1,3-dioxolane, and 2-(8-methyldodecyl)-1,3-dioxane.

B-2. Process for preparing 1,1-dialkoxy-9-methyltridecane compound (14) by coupling reaction of 1-halo-3-methylheptane compound (16) and 6,6-dialkoxyhexylmagnesium halide compound (15)

B-2-1. 1-Halo-3-methylheptane compound (16)

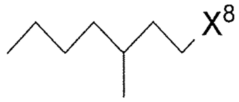

(16)

In the general formula (16), $X^8$ represents a halogen atom.

Specific examples of the halogen atom $X^8$ include a chlorine atom, a bromine atom, and an iodine atom. The halogen atom $X^8$ is preferably a bromine atom and an iodine atom. By using said bromine atom and iodine atom, a preferred reactivity may be ensured.

Specific examples of the 1-halo-3-methylheptane compound (16) include 1-chloro-3-methylheptane, 1-bromo-3-methylheptane, and 1-iodo-3-methylheptane.

The 1-halo-3-methylheptane compound (16) is preferably 1-bromo-3-methylheptane and 1-iodo-3-methylheptane. By using said 1-bromo-3-methylheptane and 1-iodo-3-methylheptane, a preferred reactivity may be ensured.

The 1-halo-3-methylheptane compound (16) may be used alone or in combination thereof, if necessary.

The 1-halo-3-methylheptane compound (16) may be a commercially available one, or may be synthesized in house.

B-2-2. 6,6-Dialkoxyhexylmagnesium halide compound (15)

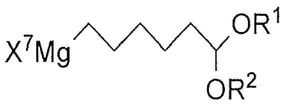

(15)

$R^1$ and $R^2$ of the general formula (15) are as defined for the general formula (14).

In the general formula (15), $X^7$ represents a halogen atom. Specific examples of the halogen atom $X^7$ include a chlorine atom, a bromine atom, and an iodine atom. The halogen atom $X^7$ is preferably a chlorine atom and a bromine atom. By using said chlorine atom and bromine atom, a preferred handling may be ensured. The halogen atom $X^7$ is more preferably a chlorine atom. By using said chlorine atom, a more preferred solidification prevention may be ensured.

Specific examples of the 6,6-dialkoxyhexylmagnesium halide compound (15) include the following compounds:

6,6-dialkoxyhexylmagnesium chloride compounds (15: $X^7$ = chlorine atom) such as 6,6-dimethoxyhexylmagnesium chloride, 6,6-diethoxyhexylmagnesium chloride, 6,6-dipropyloxyhexylmagnesium chloride, 6,6-dibutyloxyhexylmagnesium chloride, 6,6-dipentyloxyhexylmagnesium chloride, 6,6-dihexyloxyhexylmagnesium chloride, 6,6-diheptyloxyhexylmagnesium chloride, 6,6-dioctyloxyhexylmagnesium chloride, 6,6-dinonyloxyhexylmagnesium chloride, 6,6-didecyloxyhexylmagnesium chloride, [5-(1,3-dioxolan-2-yl)pentyl]magnesium chloride, and [5-(1,3-dioxan-2-yl)pentyl]magnesium chloride;

6,6-dialkoxyhexylmagnesium bromide compounds (15: $X^7$ = bromine atom) such as 6,6-dimethoxyhexylmagnesium bromide, 6,6-diethoxyhexylmagnesium bromide, 6,6-dipropyloxyhexylmagnesium bromide, 6,6-dibutyloxyhexylmagnesium bromide, 6,6-dipentyloxyhexylmagnesium bromide, 6,6-dihexyloxyhexylmagnesium bromide, 6,6-diheptyloxyhexylmagnesium bromide, 6,6-dioctyloxyhexylmagnesium bromide, 6,6-dinonyloxyhexylmagnesium bromide, 6,6-didecyloxyhexylmagnesium bromide, [5-(1,3-dioxolan-2-yl)pentyl]magnesium bromide, and [5-(1,3-dioxan-2-yl)pentyl]magnesium bromide; and

6,6-dialkoxyhexylmagnesium iodide compounds (15: $X^7$ = iodine atom) such as 6,6-dimethoxyhexylmagnesium iodide, 6,6-diethoxyhexylmagnesium iodide, 6,6-dipropyloxyhexylmagnesium iodide, 6,6-dibutyloxyhexylmagnesium iodide, 6,6-dipentyloxyhexylmagnesium iodide, 6,6-dihexyloxyhexylmagnesium iodide, 6,6-diheptyloxyhexylmagnesium iodide, 6,6-dioctyloxyhexylmagnesium iodide, 6,6-dinonyloxyhexylmagnesium iodide, 6,6-didecyloxyhexylmagnesium iodide, [5-(1,3-dioxolan-2-yl)pentyl]magnesium iodide, and [5-(1,3-dioxan-2-yl)pentyl]magnesium iodide.

The amount of the 6,6-dialkoxyhexylmagnesium halide compound (15) used in the coupling reaction is preferably 0.6 to 2.0 mol, and more preferably 0.8 to 1.4 mol, per mol of the 1-halo-3-methylheptane compound (16). By using said preferred amount and said more preferred amount, a preferred reactivity and a more preferred reactivity may be ensured.

The 6,6-dialkoxyhexylmagnesium halide compound (15) may be used alone or in combination thereof, if necessary.

The 6,6-dialkoxyhexylmagnesium halide compound (15) may be a commercially available one, or may be synthesized in house.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. The solvent is preferably hydrocarbon solvents such as toluene and xylene; ether solvents such as tetrahydrofuran, 2-methyltetrahydrofuran, and 4-methyltetrahydropyran; and acetonitrile. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. The solvent is more preferably tetrahydrofuran, 2-methyltetrahydrofuran, toluene, and xylene. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used is preferably 30 to 8,000 g, and more preferably 50 to 5,000 g, per mol of the 1-halo-3-methylheptane compound (16). By using said preferred amount and said more preferred amount, a preferred reactivity and a more preferred reactivity may be ensured.

The 6,6-dialkoxyhexylmagnesium halide compound (15) may be subjected to the coupling reaction with the 1-halo-3-methylheptane compound (16) in the presence of a catalyst, if necessary.

Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III) acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene]palladium; and nickel compounds such as nickel chloride, dichloro [1,2-bis(diphenylphosphino)ethane]nickel(II), and dichlorobis(triphenylphosphine)nickel(II). The catalyst is preferably copper compounds. By using said copper compounds, a preferred reactivity and/or economy may be ensured. The catalyst is more preferably cuprous halides such as cuprous chloride, cuprous bromide, and cuprous iodide. By using said cuprous halides such as cuprous chloride, cuprous bromide, and cuprous iodide, a more preferred reactivity and/or economy may be ensured.

The catalyst may be used alone or in combination thereof, if necessary. The catalyst may be a commercially available one.

The amount of the catalyst used is preferably 0.0001 to 1.00 mol, and more preferably 0.001 to 0.300 mol, per mol of the 1-halo-3-methylheptane compound (16). By using said preferred amount and said more preferred amount, a preferred reaction rate and post-processing and a more preferred reaction rate and post-processing may be ensured.

When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). The co-catalyst is preferably trialkyl phosphite compounds, and more preferably triethyl phosphite. By using said trialkyl phosphite compounds, a preferred reactivity may be ensured. By using said triethyl phosphite, a more preferred reactivity may be ensured.

The co-catalyst may be used alone or in combination thereof, if necessary. The co-catalyst may be a commercially available one.

The amount of the co-catalyst used is preferably 0.0001 to 1.00 mol, and more preferably 0.001 to 0.300 mol, per mol of the 1-halo-3-methylheptane compound (16). By using said preferred amount and said more preferred amount, a preferred reactivity and a more preferred reactivity may be ensured.

When the coupling reaction is carried out in the presence of a catalyst, a lithium salt may be added, if necessary. Examples of the lithium salt include lithium halides such as lithium chloride, lithium bromide, and lithium iodide; lithium nitrate; and lithium carbonate. The lithium salt is preferably lithium halides such as lithium chloride; and lithium nitrate. By

using said lithium halides such as lithium chloride; and lithium nitrate, a preferred reactivity may be ensured.

The lithium salt may be used alone or in combination thereof, if necessary. The lithium salt may be a commercially available one.

The amount of the lithium salt used in the coupling reaction is preferably 0.0001 to 1.00 mol, and more preferably 0.001 to 0.300 mol, per mol of the 1-halo-3-methylheptane compound (16). By using said preferred amount and said more preferred amount, a preferred reactivity and a more preferred reactivity may be ensured.

The reaction temperature of the coupling reaction varies, depending on the 6,6-dialkoxyhexylmagnesium halide compound (15) to be used, and is preferably -78 to 100°C, and more preferably -25 to 60°C. By using said preferred reaction temperature and said more preferred reaction temperature, a preferred reactivity and a more preferred reactivity may be ensured.

The reaction time of the coupling reaction varies, depending on the solvent to be used and/or the production scale, and is preferably 0.5 to 100 hours. By using said reaction time, a preferred reactivity may be ensured.

The preparation of 9-methyltridecanal (13) will be explained below in Section C.

C. Process for preparing 9-methyltridecanal (13) by subjecting 1,1-dialkoxy-9-methyltridecane compound (14) to hydrolysis reaction

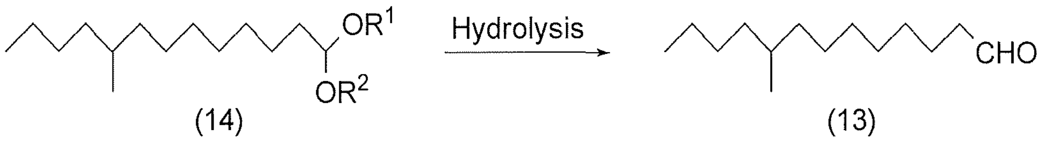


(14) (13)

C-1. Hydrolysis reaction

In the aforesaid hydrolysis reaction, the 1,1-dialkoxy-9-methyltridecane compound (14) may be used alone or in combination thereof, if necessary.

The hydrolysis reaction may be carried out, for example, with an acid and water.

Examples of the acid include inorganic acids such as hydrochloric acid and hydrobromic acid; and *p*-toluenesulfonic acid, benzenesulfonic acid, trifluoroacetic acid, acetic acid, formic acid, oxalic acid, iodotrimethylsilane, and titanium tetrachloride. The acid is preferably acetic acid, formic acid, and oxalic acid. By using said acetic acid, formic acid, and oxalic acid, a preferred reactivity may be ensured.

The acid may be used alone or in combination thereof, if necessary. The acid may be a commercially available one.

The amount of the acid used is preferably 0.01 to 10.0 mol, per mol of the 1,1-dialkoxy-9-methyltridecane compound (14).

The amount of the water used is preferably 18 to 7,000 g, and more preferably 18 to 3,000 g, per mol of the 1,1-dialkoxy-9-methyltridecane compound (14). By using said preferred amount and said more preferred amount, a preferred reactivity and a more preferred reactivity may be ensured.

In the hydrolysis reaction, a solvent may be further incorporated, if necessary, in addition to the acid or water.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, acetone, *N*,*N*'-dimethylpropylene urea (DMPU), hexamethylphosphoric triamide (HMPA), dichloromethane, and chloroform; ester solvents such as methyl acetate, ethyl acetate, n-propyl acetate, and n-butyl acetate; and alcoholic solvents such as methanol and ethanol.

The solvent may be used alone or in combination thereof, if necessary. The solvent may be a commercially available one.

The optimal solvent varies, depending on the acid to be used, and when, for example, oxalic acid is used as the acid, the acid is preferably tetrahydrofuran, 2-methyltetrahydrofuran, acetone, and γ-butyrolactone. By using said tetrahydrofuran, 2-methyltetrahydrofuran, acetone, and γ-butyrolactone, a preferred reactivity may be ensured.

The amount of the solvent used is preferably 0 to 7,000 g, and more preferably 18 to 3,000 g, per mol of the 1,1-dialkoxy-9-methyltridecane compound (14). By using said preferred amount and said more preferred amount, a preferred reactivity and a more preferred reactivity may be ensured.

The reaction temperature of the hydrolysis reaction varies, depending on the acid and/or solvent to be used, and

is preferably -15 to 180°C, and more preferably 5 to 120°C. By using said preferred reaction temperature and said more preferred reaction temperature, a preferred reactivity and a more preferred reactivity may be ensured.

The reaction time of the hydrolysis reaction varies, depending on the acid to be used, the solvent to be used, and/or the production scale, and is preferably 0.5 to 100 hours.

The preparation of 10-methyl-2-tetradecanol (11) will be explained below in Section D.

D. Process for preparing 10-methyl-2-tetradecanol (11) by subjecting 9-methyltridecanal (13) to addition reaction with nucleophilic reagent, methyl (12)

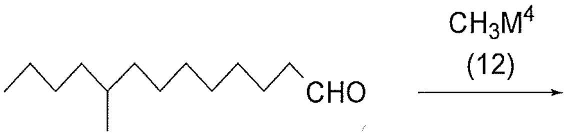


(13) (11)

D-1. Nucleophilic reagent, methyl (12)

In the general formula (12), $M^4$ represents Li and $MgZ^4$, and $Z^4$ represents a halogen atom or a methyl group. Examples of the halogen atom $Z^4$ include a chlorine atom, a bromine atom, and an iodine atom.

Specific examples of the nucleophilic reagent, methyl (12), include methyllithium; and methylmagnesium halide reagents (Grignard reagents) such as methylmagnesium chloride, methylmagnesium bromide, and methylmagnesium iodide. The nucleophilic reagent is preferably a methylmagnesium halide reagent. By using said methylmagnesium halide reagent, a preferred ease of preparation (availability) may be ensured.

The nucleophilic reagent, methyl (12), may be used alone or in combination thereof, if necessary. The nucleophilic reagent, methyl (12), may be a commercially available one, or may be synthesized in house.

D-2

The addition reaction of the nucleophilic reagent, methyl (12), with 9-methyltridecanal (13) will be described in detail below.

The amount of the nucleophilic reagent, methyl (12), used in the addition reaction is preferably 0.8 to 1.2 mol, per mol of 9-methyltridecanal (13). By using said preferred amount, preferred economy may be ensured.

A solvent may be incorporated in the addition reaction, if necessary. Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N'*-dimethylpropylene urea (DMPU), hexamethylphosphoric triamide (HMPA), dichloromethane, and chloroform. The solvent is preferably hydrocarbon solvents such as toluene and xylene; ether solvents such as tetrahydrofuran, 2-methyltetrahydrofuran, and 4-methyltetrahydropyran; and acetonitrile. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. The solvent is more preferably tetrahydrofuran and 2-methyltetrahydrofuran. By using said tetrahydrofuran and 2-methyltetrahydrofuran, a more preferred reactivity may be ensured.

The solvent may be used alone or in combination thereof, if necessary. The solvent may be a commercially available one.

The amount of the solvent used is preferably 30 to 5,000 g, and more preferably 50 to 3,000 g, per mol of 9-methyltridecanal (13). By using said preferred amount and said more preferred amount, a preferred reactivity and a more preferred reactivity may be ensured.

The reaction temperature of the addition reaction varies, depending on the nucleophilic reagent, methyl (12), to be used, and is preferably -5 to 120°C, more preferably 20 to 100°C, and even more preferably 40 to 80°C. By using said preferred reaction temperature, said more preferred reaction temperature, and said even more preferred reaction temperature, a preferred reaction yield, a more preferred reaction yield, and an even more preferred reaction yield may be ensured.

The reaction time of the addition reaction varies, depending on the solvent to be used and/or the production scale,

and is preferably 0.5 to 100 hours. By using said reaction time, a preferred reactivity may be ensured.

The preparation of the 2-halo-10-methyltetradecane compound (9) will be explained below in Section E.

E. Process for preparing 2-halo-10-methyltetradecane compound (9) by halogenating 10-methyl-2-tetradecanol (11)

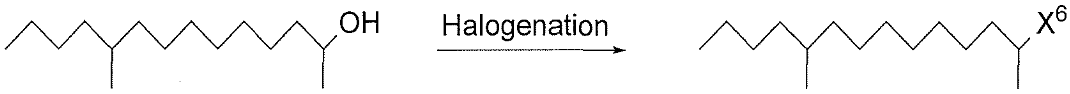


(11) (9)

E-1. Halogenation reaction

The halogenation reaction may be carried out by, for example, a process of tosylating a hydroxy group with a *p*-toluenesulfonyl halide compound, and then halogenating with a metal salt, lithium halide compound; or a process of directly halogenating a hydroxy group with a halogenating agent.

Examples of the halogenating agent include halogens such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; *p*-toluenesulfonyl halide compounds such as *p*-toluenesulfonyl chloride, *p*-toluenesulfonyl bromide, and *p*-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, *tert*-butyldimethylsilyl chloride, *tert*-butyldimethylsilyl bromide, and *tert*-butyldimethylsilyl iodide; oxalyl halide compounds such as oxalyl chloride, oxalyl bromide, and oxalyl iodide; and *N*-halosuccinimide compounds such as *N*-chlorosuccinimide, *N*-bromosuccinimide, and *N*-iodosuccinimide. The halogenating agent is preferably a methanesulfonyl halide compound, a benzenesulfonyl halide compound, a *p*-toluenesulfonyl halide compound, and a thionyl halide compound. By using said methanesulfonyl halide compound, benzenesulfonyl halide compound, *p*-toluenesulfonyl halide compound, and thionyl halide compound, a preferred suppression of side reactions may be ensured. The halogenating agent is more preferably a methanesulfonyl halide compound, a benzenesulfonyl halide compound, and a thionyl halide compound. By using said methanesulfonyl halide compound, benzenesulfonyl halide compound, and thionyl halide compound, a more preferred suppression of side reactions may be ensured.

The halogenating agent may be used alone or in combination thereof, if necessary. The halogenating agent may be a commercially available one.

The amount of the halogenating agent used is preferably 0.8 to 5.0 mol, and more preferably 1.0 to 2.5 mol, per mol of 10-methyl-2-tetradecanol (11). By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, *N*,*N*-diisopropylethylamine, piperidine, pyrrolidine, pyridine, lutidine, 4-dimethylaminopyridine, *N*,*N*-dimethylaniline, *N*,*N*-diethylaniline, and 1,8-diazabicyclo[5.4.0]-7-undecene (DBU).

When the halogenating agent is a methanesulfonyl halide compound, a benzenesulfonyl halide compound, and a *p*-toluenesulfonyl halide compound, the base is preferably amines, and more preferably pyridine, lutidine, and 4-dimethylaminopyridine.

When the halogenating agent is a thionyl halide compound, the base is preferably amines, and more preferably trialkylamines such as triethylamine.

The base may be used alone or in combination thereof, if necessary. The base may be a commercially available one.

The amount of the base used is preferably 0 to 8.0 mol, and more preferably 0 to 3.0 mol, per mol of 10-methyl-2-tetradecanol (11). By using said preferred amount and said more preferred amount, a preferred yield and/or economy and a more preferred yield and/or economy may be ensured.

A metal salt may be added in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

For example, when the halogenation with the metal salt, lithium halide compound, is carried out after the tosylation, the reaction is carried out with lithium salts such as lithium chloride, lithium bromide, and lithium iodide.

The metal salt may be used alone or in combination thereof, if necessary. The metal salt may be a commercially available one.

The amount of the metal salt used is preferably 0 to 30.0 mol, and more preferably 0 to 5.0 mol, per mol of 10-methyl-2-tetradecanol (11). By using said preferred amount and said more preferred amount, a preferred reactivity and a more preferred reactivity may be ensured.

Although the metal salt increases the concentration of halide ions in the reaction system to thereby enhance the reactivity, it is preferred not to incorporate the metal salt. By not incorporating the metal salt, preferred economy and/or environmental protection may be ensured.

A solvent may be incorporated in the halogenation reaction, if necessary.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone(GBL), acetonitrile, acetone, *N*,*N'*-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. The solvent is preferably 2-methyltetrahydrofuran, 4-methyltetrahydropyran, dichloromethane, chloroform, γ-butyrolactone, *N*-methylpyrrolidone, *N*,*N*-dimethylformamide, *N*,*N*-dimethylacetamide, and acetonitrile. By using said 2-methyltetrahydrofuran, 4-methyltetrahydropyran, dichloromethane, chloroform, γ-butyrolactone, *N*-methylpyrrolidone, *N,N*-dimethylformamide, *N*,*N*-dimethylacetamide, and acetonitrile, a preferred reactivity may be ensured. The solvent is more preferably 2-methyltetrahydrofuran, γ-butyrolactone, and acetonitrile. By using said 2-methyltetrahydrofuran, γ-butyrolactone, and acetonitrile, more preferably safety may be ensured.

The solvent may be used alone or in combination thereof, if necessary. The solvent may be a commercially available one.

The amount of the solvent used in the halogenation reaction is preferably 0 to 3,000 g, and more preferably 0 to 800 g, per mol of 10-methyl-2-tetradecanol (11).

The solvent may occupy part of the reactor space, which reduces the space for the starting materials, and reduces productivity. Therefore, the reaction may be carried out without a solvent, or with the base as the solvent.

The reaction temperature of the halogenation reaction varies, depending on the halogenating agent to be used, and is preferably 5 to 180°C, and more preferably 20 to 120°C. By using said preferred reaction temperature and said more preferred reaction temperature, a preferred reactivity and a more preferred reactivity may be ensured.

The reaction time of the halogenation reaction varies, depending on the halogenating agent to be used and/or the production scale, and is preferably 0.5 to 100 hours. By using said reaction time, a preferred reactivity may be ensured.

E-2. 2-Halo-10-methyltetradecane compound (9)

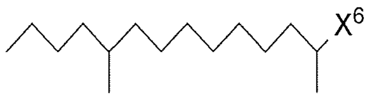

(9)

wherein $X^6$ represents a halogen atom. Examples of the halogen atom $X^6$ include a chlorine atom, a bromine atom, and an iodine atom.

Specific examples of the 2-halo-10-methyltetradecane compound (9) include 2-chloro-10-methyltetradecane, 2-bromo-10-methyltetradecane, and 2-iodo-10-methyltetradecane.

The preparation of 3,11-dimethylpentadecanol (8) will be explained below in Section F.

F. Process for preparing 3,11-dimethylpentadecanol (8) by converting 2-halo-10-methyltetradecane compound (9) into nucleophilic reagent, 1,9-dimethyltridecyl (10), and then subjecting nucleophilic reagent, 1,9-dimethyltridecyl (10), to homologation reaction with ethylene oxide

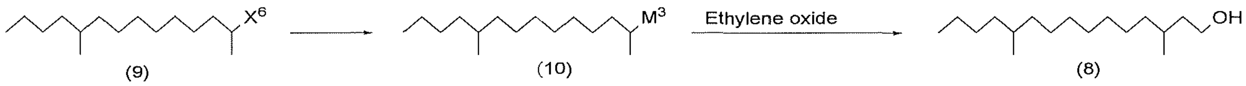


(9) (10) (8)

F-1. Nucleophilic reagent, 1,9-dimethyltridecyl (10)

The nucleophilic reagent, 1,9-dimethyltridecyl (10), may be prepared in a conventional method or a process described below.

A process for preparing a 1,9-dimethyltridecylmagnesium halide reagent (10: $M^3$ = $MgZ^3$) will be described in detail below as an example of the nucleophilic reagent, 1,9-dimethyltridecyl (10). The 1,9-dimethyltridecylmagnesium halide reagent (10: $M^3$ = $MgZ^3$) may be prepared by, for example, reacting the aforesaid 2-halo-10-methyltetradecane compound (9) with magnesium in a solvent, as shown in the following chemical reaction formula.

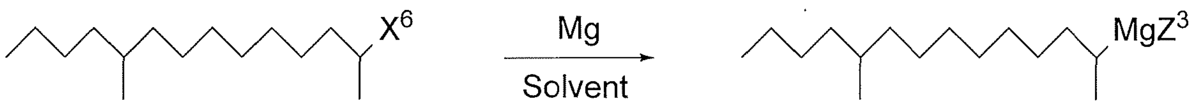


(9) (10:$M^3$=$MgZ^3$)

The 1,9-dimethyltridecylmagnesium halide reagent (10: $M^3$ = $MgZ^3$) is a Grignard reagent, wherein $Z^3$ is the same as $X^6$, and represents a halogen atom. Examples of the halogen atom $X^6$ include a chlorine atom, a bromine atom, and an iodine atom.

The 2-halo-10-methyltetradecane compound (9) may be used alone or in combination thereof, if necessary.

The amount of magnesium used is preferably 1.0 to 2.0 gram atoms, per mol of the 2-halo-10-methyltetradecane compound (9). By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. The solvent is preferably ether solvents such as tetrahydrofuran, 2-methyltetrahydrofuran, diethyl ether, and 4-methyltetrahydropyran. By using said ether solvents such as tetrahydrofuran, 2-methyltetrahydrofuran, diethyl ether, and 4-methyltetrahydropyran, a preferred reaction rate of the Grignard reagent formation may be ensured. The solvent is more preferably tetrahydrofuran and 2-methyltetrahydrofuran. By using said tetrahydrofuran and 2-methyltetrahydrofuran, a more preferred reaction rate of the Grignard reagent formation may be ensured.

The solvent may be used alone or in combination thereof, if necessary. The solvent may be a commercially available one.

The amount of the solvent used is preferably 30 to 5,000 g, and more preferably 50 to 3,000 g, per mol of the 2-halo-10-methyltetradecane compound (9). By using said preferred amount and said more preferred amount, a preferred reactivity and a more preferred reactivity may be ensured.

The reaction temperature of the aforesaid reaction with magnesium varies, depending on the solvent to be used, and is preferably 30 to 120°C. By using said preferred reaction temperature, a preferred reactivity may be ensured.

The reaction time of the aforesaid reaction with magnesium varies, depending on the solvent to be used and/or the production scale, and is preferably 0.5 to 100 hours. By using said preferred reaction time, a preferred reactivity may be ensured.

F-2. Process for preparing 3,11-dimethylpentadecanol (8) by subjecting nucleophilic reagent, 1,9-dimethyltridecyl (10), to homologation reaction with ethylene oxide

The amount of ethylene oxide used is preferably 1.0 to 10.0 mol, and more preferably 1.0 to 5.0 mol, per mol of the 2-halo-10-methyltetradecane compound (9). By using said preferred amount and said more preferred amount, a preferred

reactivity and a more preferred reactivity may be ensured.

A solvent may be incorporated in the homologation reaction mentioned above, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N,N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. The solvent is preferably ether solvents such as diethyl ether, tetrahydrofuran, 2-methyltetrahydrofuran, and 4-methyltetrahydropyran; and hydrocarbon solvents such as toluene and xylene. By using said ether solvents such as diethyl ether, tetrahydrofuran, 2-methyltetrahydrofuran, and 4-methyltetrahydropyran; and hydrocarbon solvents such as toluene and xylene, a preferred reactivity may be ensured.

The solvent may be used alone or in combination thereof, if necessary. The solvent may be a commercially available one.

The amount of the solvent used is preferably 20 to 7,000 g, and more preferably 50 to 3,000 g, per mol of the 2-halo-10-methyltetradecane compound (9). By using said preferred amount and said more preferred amount, a preferred reactivity and a more preferred reactivity may be ensured.

The reaction temperature of the homologation reaction varies, depending on the nucleophilic reagent, 1,9-dimethyltridecyl (10), to be used and/or the solvent to be used, and is preferably -40 to 180°C, more preferably -25 to 100°C, and even more preferably -10 to 70°C. By using said preferred reaction temperature, said more preferred reaction temperature, and said even more preferred reaction temperature, a preferred reactivity, a more preferred reactivity, and an even more preferred reactivity may be ensured.

The reaction time of the homologation reaction varies, depending on the nucleophilic reagent to be used, the solvent to be used, and/or the production scale, and is preferably 0.5 to 100 hours. By using said preferred reaction time, a preferred reactivity may be ensured.

The preparation of the 1-halo-3,11-dimethylpentadecane compound (5) will be explained below in Section G.

G. Process for preparing 1-halo-3,11-dimethylpentadecane compound (5) by halogenating 3,11-dimethylpentadecanol (8)

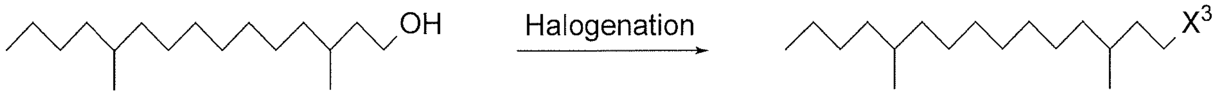


(8) (5)

G-1. Halogenation reaction

The halogenation reaction of 3,11-dimethylpentadecanol (8) is similar to E-1 above. In Section G, the amounts of the halogenating agent, the base, the metal salt, and the solvent used are amounts per mol of 3,11-dimethylpentadecanol (8).

The preparation of the 1-halo-2,6,14-trimethyloctadecane compound (1) will be explained below in Section H.

H. Process for preparing 1-halo-2,6,14-trimethyloctadecane compound (1) by converting 1-halo-3,11-dimethylpentadecane compound (5) into nucleophilic reagent, 3,11-dimethylpentadecyl (6), and then subjecting nucleophilic reagent, 3,11-dimethylpentadecyl, to coupling reaction with 1,3-dihalo-2-methylpropane compound (7)

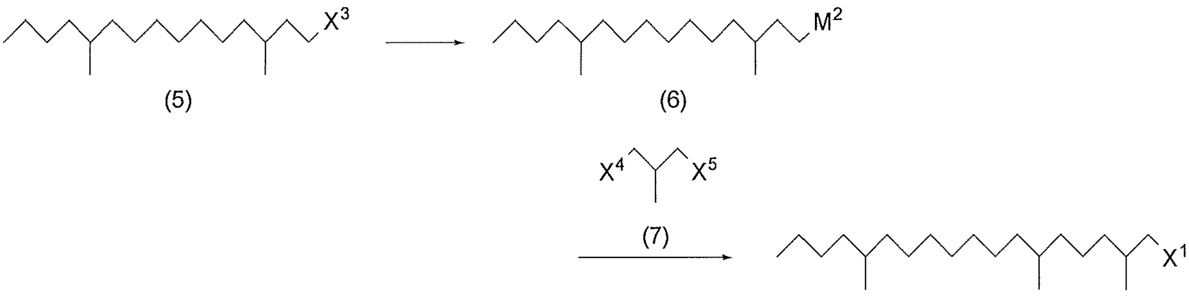

(5) (6) (7) (1)

H-1. Nucleophilic reagent, 3,11-dimethylpentadecyl (6)

The nucleophilic reagent, 3,11-dimethylpentadecyl (6), may be prepared in a conventional method or a process described below.

A process for preparing a 3,11-dimethylpentadecylmagnesium halide reagent (6: $M^2 = MgZ^2$) as an example of the nucleophilic reagent, 3,11-dimethylpentadecyl (6), will be described in detail below. The 3,11-dimethylpentadecylmagnesium halide reagent (6: $M^2 = MgZ^2$) may be prepared by, for example, reacting the aforesaid 1-halo-3,11-dimethylpentadecane compound of the following general formula (5) with magnesium in a solvent, as shown in the following chemical reaction formula.

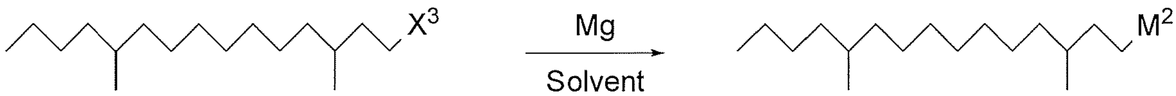


(5) (6:$M^2$=$MgZ^2$)

The 3,11-dimethylpentadecylmagnesium halide reagent (6: $M^2 = MgZ^2$) is a Grignard reagent, wherein $Z^2$ is the same as $X^3$, and represents a halogen atom. Examples of the halogen atom $X^6$ include a chlorine atom, a bromine atom, and an iodine atom.

The 1-halo-3,11-dimethylpentadecane compound (5) may be used alone or in combination thereof, if necessary. The 1-halo-3,11-dimethylpentadecane compound (5) may be a commercially available one, or may be synthesized in house.

The amount of magnesium used is preferably 1.0 to 2.0 gram atoms, per mol of the 1-halo-3,11-dimethylpentadecane compound (5). By using said preferred amount, a preferred completion of the reaction may be ensured.

The solvent, as well as the amount used, reaction temperature, and reaction time of the solvent, are similar to the solvent and the amount used, reaction temperature, and reaction time of the solvent according to F-1.

H-2. 1,3-Dihalo-2-methylpropane compound (7)

$X^4$ and $X^5$ of the general formula (7) represent, independently of each other, a halogen atom. Examples of the halogen atoms $X^4$ and $X^5$ include a chlorine atom, a bromine atom, and an iodine atom.

Examples of combinations of $X^4$ and $X^5$ include a chlorine atom and a chlorine atom, a bromine atom and a chlorine atom, a chlorine atom and an iodine atom, a bromine atom and a bromine atom, a bromine atom and an iodine atom, and an iodine atom and an iodine atom.

Specific examples of the 1,3-dihalo-2-methylpropane compound (7) include 1,3-dichloro-2-methylpropane, 1,3-dibromo-2-methylpropane, 1,3-diiodo-2-methylpropane, 1-bromo-3-chloro-2-methylpropane, 1-chloro-3-iodo-2-methylpropane, and 1-bromo-3-iodo-2-methylpropane.

The 1,3-dihalo-2-methylpropane compound (7) is more preferably 1-bromo-3-chloro-2-methylpropane, 1-chloro-3-iodo-2-methylpropane, and 1-bromo-3-iodo-2-methylpropane. By using said 1-bromo-3-chloro-2-methylpropane, 1-chloro-3-iodo-2-methylpropane, and 1-bromo-3-iodo-2-methylpropane, a more preferred yield may be ensured.

The 1,3-dihalo-2-methylpropane compound (7) may be used alone or in combination thereof, if necessary. The 1,3-dihalo-2-methylpropane compound (7) may be a commercially available one, or may be synthesized in house.

The 1,3-dihalo-2-methylpropane compound (7) may be synthesized by, for example, halogenating 2-methyl-1,3-propanediol.

H-3. Coupling reaction of nucleophilic reagent, 3,11-dimethylpentadecyl (6), with 1,3-dihalo-2-methylpropane compound (7)

The amount of the nucleophilic reagent, 3,11-dimethylpentadecyl (6), used in the coupling reaction is preferably 0.8 to 1.4 mol, per mol of the 1,3-dihalo-2-methylpropane compound (7). By using said preferred amount, preferred economy may be ensured.
A solvent may be incorporated in the coupling reaction, if necessary. Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N*-dimethylacetamide (DMAC), *N*-methylpyrrolidone (NMP), dimethyl sulfoxide (DMSO), γ-butyrolactone(GBL), acetonitrile, acetone, *N,N'*-dimethylpropylene urea (DMPU), hexamethylphosphoric triamide (HMPA), dichloromethane, and chloroform. The solvent is preferably toluene, tetrahydrofuran, 2-methyltetrahydrofuran, 4-methyltetrahydropyran, and acetonitrile. By using said toluene, tetrahydrofuran, 2-methyltetrahydrofuran, 4-methyltetrahydropyran, and acetonitrile, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.
The solvent may be used alone or in combination thereof, if necessary. The solvent may be a commercially available one.
The amount of the solvent used is preferably 30 to 5,000 g, and more preferably 50 to 3,000 g, per mol of the 1,3-dihalo-2-methylpropane compound (7). By using said preferred amount and said more preferred amount, a preferred reactivity and a more preferred reactivity may be ensured.
The coupling reaction may be carried out in the presence of a catalyst, if necessary. Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III) acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene] palladium; and nickel compounds such as nickel chloride, dichloro[1,2-bis(diphenylphosphino)ethane]nickel(II), and dichlorobis(triphenylphosphine)nickel(II). The catalyst is preferably a copper compound. By using said copper compound, a preferred reactivity and/or economy may be ensured. The catalyst is more preferably cuprous halides such as cuprous chloride, cuprous bromide, and cuprous iodide. By using said cuprous halides such as cuprous chloride, cuprous bromide, and cuprous iodide, a more preferred reactivity and/or economy may be ensured. The catalyst is most preferably cuprous iodide. By using said cuprous iodide, a most preferred reactivity and/or economy may be ensured.
The catalyst may be used alone or in combination thereof, if necessary. The catalyst may be a commercially available one.
The amount of the catalyst used is preferably 0.0001 to 1.00 mol, and more preferably 0.001 to 0.300 mol, per mol of the 1,3-dihalo-2-methylpropane compound (7). By using said preferred amount and said more preferred amount, a preferred reaction rate and/or post-processing and a more preferred reaction rate and/or post-processing may be ensured.
When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). The co-catalyst is preferably trialkyl phosphite. By using said trialkyl phosphite, a preferred reactivity may be ensured. The co-catalyst is more preferably triethyl phosphite. By using said triethyl phosphite, a more preferred reactivity may be ensured.
The co-catalyst may be used alone or in combination thereof, if necessary. The co-catalyst may be a commercially available one.
The amount of the co-catalyst used is preferably 0.0001 to 1.00 mol, and more preferably 0.001 to 0.300 mol, per mol of the 1,3-dihalo-2-methylpropane compound (7). By using said preferred amount and said more preferred amount, a preferred reactivity and a more preferred reactivity may be ensured.
When the coupling reaction is carried out in the presence of a catalyst, a lithium salt may be added, if necessary. Examples of the lithium salt include lithium halides such as lithium chloride, lithium bromide, and lithium iodide; lithium nitrate; and lithium carbonate. The lithium salt is preferably lithium halides such as lithium chloride; and lithium nitrate. By using said lithium halides such as lithium chloride; and lithium nitrate, a preferred reactivity may be ensured.
The lithium salt may be used alone or in combination thereof, if necessary. The lithium salt may be a commercially available one.
The amount of the lithium salt used in the coupling reaction is preferably 0.005 to 0.250 mol, per mol of the 1,3-dihalo-2-methylpropane compound (7). By using said preferred amount, a preferred reactivity may be ensured.

The reaction temperature of the coupling reaction varies, depending on the nucleophilic reagent, 3,11-dimethylpentadecyl (6), to be used, and is preferably -78 to 70°C, and more preferably -20 to 35°C. By using said preferred reaction temperature and said more preferred reaction temperature, a preferred reactivity and a more preferred reactivity may be ensured.

The reaction time of the coupling reaction varies, depending on the solvent to be used and/or the production scale, and is preferably 0.5 to 100 hours. By using said preferred reaction time, a preferred reactivity may be ensured.

When $X^4$ and $X^5$ of the general formula (7) are different from each other, appropriate selection of the aforesaid catalyst or reaction temperature allows a more reactive halogen atom to react in the coupling reaction. For example, when a combination of $X^4$ and $X^5$, which are different from each other in the 1,3-dihalo-2-methylpropane compound (7), is a combination of a chlorine atom and a bromine atom or a combination of a chlorine atom and an iodine atom, $X^1$ in the 1-halo-2,6,14-trimethyloctadecane compound (1) would be a chlorine atom. When a combination of $X^4$ and $X^5$, which are different from each other in the 1,3-dihalo-2-methylpropane compound (7), is a combination of a bromine atom and an iodine atom, $X^1$ in the 1-halo-2,6,14-trimethyloctadecane compound (1) would be a bromine atom.

The process for preparing the 5,13,17-trimethylalkane compound (4) with the 1-halo-2,6,14-trimethyloctadecane compound (1) will be explained below in Section I.

I-1

The 5,13,17-trimethylalkane compound (4) may be prepared by converting the 1-halo-2,6,14-trimethyloctadecane compound (1) into the nucleophilic reagent, 2,6,14-trimethyloctadecyl compound (2), and then subjecting the nucleophilic reagent, 2,6,14-trimethyloctadecyl compound (2), to a coupling reaction with the electrophilic alkyl reagent (3).

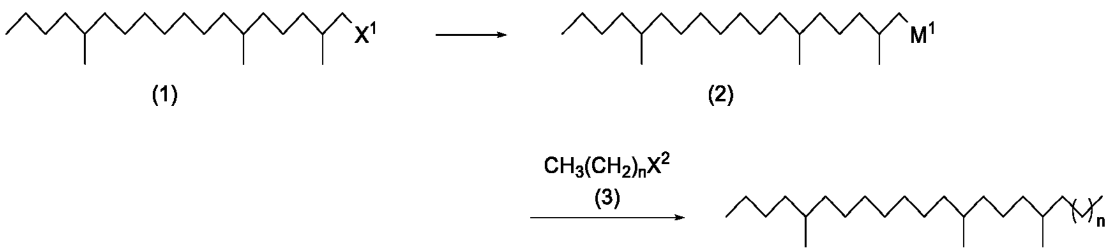


(1) (2) (4)

I-2. Process for preparing nucleophilic reagent, 2,6,14-trimethyloctadecyl compound (2)

The nucleophilic reagent, 2,6,14-trimethyloctadecyl compound (2), may be prepared in a conventional method or a process described below.

I-3

A process for preparing a 2,6,14-trimethyloctadecenylmagnesium halide reagent (2: $M^1 = MgZ^1$) as an example of the nucleophilic reagent, 2,6,14-trimethyloctadecyl compound (2), will be described in detail below.

The 2,6,14-trimethyloctadecenylmagnesium halide reagent (2: $M^1 = MgZ^1$) may be prepared by, for example, reacting the aforesaid 1-halo-2,6,14-trimethyloctadecane compound (1) with magnesium in a solvent, as shown in the following chemical reaction formula.

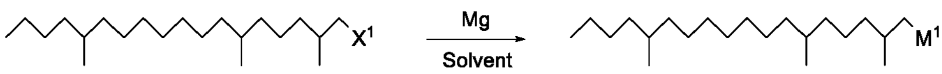


(1) (2:$M^1$=$MgZ^1$)

The 2,6,14-trimethyloctadecenylmagnesium halide reagent (2: $M^1 = MgZ^1$) is a Grignard reagent, wherein $Z^1$ is the same as $X^1$, and represents a halogen atom. Examples of the halogen atom $X^1$ include a chlorine atom, a bromine atom, and an iodine atom as mentioned above.

The 1-halo-2,6,14-trimethyloctadecane compound (1) may be used alone or in combination thereof, if necessary. The 1-halo-2,6,14-trimethyloctadecane compound (1) may be prepared by, for example, the aforesaid processes of Sections B to H.

The amount of magnesium used is preferably 1.0 to 2.0 gram atoms, per mol of the 1-halo-2,6,14-trimethyloctadecane compound (1). By using said preferred amount, a preferred completion of the reaction may be ensured.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N*'-dimethylpropylene urea (DMPU), hexamethylphosphoric triamide (HMPA), dichloromethane, and chloroform. The solvent is preferably ether solvents such as tetrahydrofuran, 2-methyltetrahydrofuran, diethyl ether, and 4-methyltetrahydropyran. By using said ether solvents such as tetrahydrofuran, 2-methyltetrahydrofuran, diethyl ether, and 4-methyltetrahydropyran, a preferred reaction rate of the Grignard reagent formation may be ensured. The solvent is more preferably tetrahydrofuran and 2-methyltetrahydrofuran. By using said tetrahydrofuran and 2-methyltetrahydrofuran, a more preferred reaction rate of the Grignard reagent formation may be ensured.

The solvent may be used alone or in combination thereof, if necessary. The solvent may be a commercially available one.

The amount of the solvent used is preferably 30 to 5,000 g, and more preferably 50 to 3,000 g, per mol of the 1-halo-2,6,14-trimethyloctadecane compound (1). By using said preferred amount and said more preferred amount, a preferred reactivity and a more preferred reactivity may be ensured.

The reaction temperature of the aforesaid reaction with magnesium varies, depending on the solvent to be used, and is preferably 30 to 120°C. By using said preferred reaction temperature, a preferred reactivity may be ensured.

The reaction time of the aforesaid reaction with magnesium varies, depending on the solvent to be used and/or the production scale, and is preferably 0.5 to 100 hours. By using said preferred reaction time, a preferred reactivity may be ensured.

I-4. Electrophilic alkyl reagent (3)

In the general formula (3), $X^2$ represents a halogen atom or a *p*-toluenesulfonyloxy group ($CH_3$-$C_6H_6$-$SO_2$-O(TsO) group), and *n* represents an integer of 14 to 18. Examples of the halogen atom $X^2$ include a chlorine atom, a bromine atom, and an iodine atom. A bromine atom and an iodine atom are particularly preferred.

Specific examples of the electrophilic alkyl reagent (3) include the following compounds:

1-halopentadecane compounds such as 1-chloropentadecane, 1-bromopentadecane, and 1-iodopentadecane;
pentadecyl *p*-toluenesulfonate compounds such as pentadecyl *p*-toluenesulfonate;
1-halohexadecane compounds such as 1-chlorohexadecane, 1-bromohexadecane, and 1-iodohexadecane;
hexadecyl *p*-toluenesulfonate compounds such as hexadecyl*p*-toluenesulfonate;
1-haloheptadecane compounds such as 1-chloroheptadecane, 1-bromoheptadecane, and 1-iodoheptadecane;
heptadecyl *p*-toluenesulfonate compounds such as heptadecyl *p*-toluenesulfonate;
1-halooctadecane compounds such as 1-chlorooctadecane, 1-bromooctadecane, and 1-iodooctadecane;
octadecyl *p*-toluenesulfonate compounds such as octadecyl *p*-toluenesulfonate;
1-halononadecane compounds such as 1-chlorononadecane, 1-bromononadecane, and 1-iodononadecane; and
nonadecyl *p*-toluenesulfonate compounds such as nonadecyl*p*-toluenesulfonate.

The electrophilic alkyl reagent (3) is preferably 1-halopentadecane compounds, 1-haloheptadecane compounds, and 1-halononadecane compounds. By using said 1-halopentadecane compounds, 1-haloheptadecane compounds, and 1-halononadecane compounds, a preferred synthesis of the nest mate recognition pheromone of the Argentine ant may be ensured.

The electrophilic alkyl reagent (3) may be used alone or in combination thereof, if necessary. The electrophilic alkyl reagent (3) may be a commercially available one, or may be synthesized in house.

The electrophilic alkyl reagent (3) may be synthesized by, for example, halogenating 1-alkanol or by tosylating 1-alkanol with *p*-toluenesulfonyl chloride in the presence of a base.

I-5. Coupling reaction of nucleophilic reagent, 2,6,14-trimethyloctadecyl compound (2), with electrophilic alkyl reagent (3)

Similarly to the electrophilic alkyl reagent (3) wherein $X^2$ is a halogen atom, the electrophilic alkyl reagent (3) wherein $X^2$ is a *p*-toluenesulfonyloxy group may be incorporated in the aforesaid coupling reaction. This is because a *p*-toluenesulfonyloxy group is a good leaving group, similarly to a halogen atom such as a bromine atom (see, for example, Non-Patent Literature 4 above, page 2127, "Table 1", and related descriptions).

The amount of the nucleophilic reagent, 2,6,14-trimethyloctadecyl compound (2), used in the coupling reaction is

preferably 0.8 to 1.4 mol, per mol of the electrophilic alkyl reagent (3). By using said preferred amount, preferred economy may be ensured.

A solvent may be incorporated in the coupling reaction, if necessary. Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. The solvent is preferably toluene, tetrahydrofuran, 2-methyltetrahydrofuran, 4-methyltetrahydropyran, and acetonitrile. By using said toluene, tetrahydrofuran, 2-methyltetrahydrofuran, 4-methyltetrahydropyran, and acetonitrile, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used is preferably 30 to 5,000 g, and more preferably 50 to 3,000 g, per mol of the electrophilic alkyl reagent (3). By using said preferred amount and said more preferred amount, a preferred reactivity and a more preferred reactivity may be ensured.

The coupling reaction may be carried out in the presence of a catalyst, if necessary. Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III) acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene] palladium; and nickel compounds such as nickel chloride, dichloro[1,2-bis(diphenylphosphino)ethane]nickel(II), and dichlorobis(triphenylphosphine)nickel(II). The catalyst is preferably a copper compound. By using said copper compound, a preferred reactivity and/or economy may be ensured. The catalyst is more preferably cuprous halides such as cuprous chloride, cuprous bromide, and cuprous iodide. By using said cuprous halides such as cuprous chloride, cuprous bromide, and cuprous iodide, a more preferred reactivity and/or economy may be ensured. The catalyst is most preferably cuprous chloride. By using said cuprous chloride, a most preferred reactivity and/or economy may be ensured.

The catalyst may be used alone or in combination thereof, if necessary. The catalyst may be a commercially available one.

The amount of the catalyst used is preferably 0.0001 to 1.00 mol, and more preferably 0.001 to 0.300 mol, per mol of the electrophilic alkyl reagent (3). By using said preferred amount and said more preferred amount, a preferred reaction rate and/or post-processing and a more preferred reaction rate and/or post-processing may be ensured.

When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). The co-catalyst is preferably trialkyl phosphite. By using said trialkyl phosphite, a preferred reactivity may be ensured. The co-catalyst is more preferably triethyl phosphite. By using said triethyl phosphite, a more preferred reactivity may be ensured.

The co-catalyst may be used alone or in combination thereof, if necessary. The co-catalyst may be a commercially available one.

The amount of the co-catalyst used is preferably 0.0001 to 1.00 mol, and more preferably 0.001 to 0.300 mol, per mol of the electrophilic alkyl reagent (3). By using said preferred amount and said more preferred amount, a preferred reactivity and a more preferred reactivity may be ensured.

When the coupling reaction is carried out in the presence of a catalyst, a lithium salt may be added, if necessary. Examples of the lithium salt include lithium halides such as lithium chloride, lithium bromide, and lithium iodide; lithium nitrate; and lithium carbonate. The lithium salt is preferably lithium halides such as lithium chloride; and lithium nitrate. By using said lithium halides such as lithium chloride; and lithium nitrate, a preferred reactivity may be ensured.

The lithium salt may be used alone or in combination thereof, if necessary. The lithium salt may be a commercially available one.

The amount of the lithium salt used in the coupling reaction is preferably 0.0001 to 1.00 mol, and more preferably 0.001 to 0.300 mol, per mol of the electrophilic alkyl reagent (3). By using said preferred amount and said more preferred amount, a preferred reactivity and a more preferred reactivity may be ensured.

The reaction temperature of the coupling reaction varies, depending on the nucleophilic reagent, 2,6,14-trimethyloctadecyl compound (2), to be used, and is preferably -78 to 100°C, and more preferably -20 to 70°C. By using

said preferred reaction temperature and said more preferred reaction temperature, a preferred reactivity and a more preferred reactivity may be ensured. The reaction temperature is even more preferably 15 to 50°C. The reaction temperature of 15 to 50°C is even more preferred in view of melting point of the 5,13,17-trimethylalkane compound (4), which is a product.

The reaction time of the coupling reaction varies, depending on the solvent to be used and/or the production scale, and is preferably 0.5 to 100 hours. By using said preferred reaction time, a preferred reactivity may be ensured.

Specific examples of the 5,13,17-trimethylalkane compound (4) include 5,13,17-trimethyltritriacontane ($n$ = 14), 5,13,17-trimethyltetratriacontane ($n$ = 15), 5,13,17-trimethylpentatriacontane ($n$ = 16), 5,13,17-trimethylhexatriacontane ($n$ = 17), and 5,13,17-trimethylheptatriacontane ($n$ = 18).

Other than the preparation process of I above, the 5,13,17-trimethylalkane compound (4) may be prepared by subjecting a nucleophilic reagent, linear alkyl, having 15 to 19 carbon atoms, and specifically alkyllithium compounds such as pentadecalithium, heptadecalithium, and nonadecalithium; alkylmagnesium chloride compounds such as pentadecamagnesium chloride, heptadecamagnesium chloride, and nonadecamagnesium chloride; alkylmagnesium bromide compounds such as pentadecamagnesium bromide, heptadecamagnesium bromide, and nonadecamagnesium bromide; and alkylmagnesium iodide compounds such as pentadecamagnesium iodide, heptadecamagnesium iodide, and nonadecamagnesium iodide, to a coupling reaction with the 1-halo-2,6,14-trimethyloctadecane compound (1).

Thus, the nest mate recognition pheromone of the Argentine ant, the 5,13,17-trimethylalkane compound (4), may be efficiently prepared with fewer steps from the synthetic intermediate, the 1-halo-2,6,14-trimethyloctadecane compound (1).

EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5 or DB-WAX, 0.25 μm x 0.25 mmϕ x 30 m; carrier gas: He (1.55 mL/min), detector: FID; column temperature: 150°C, elevated in a rate of 5°C/min, and up to 230°C.

The yield was calculated according to the following equation in consideration of purities (% GC) of a starting material and a product.

Yield (%) = {[(weight of a product obtained by a reaction × % GC)/molecular weight of a product] ÷ [(weight of a starting material in a reaction × % GC)/molecular weight of a starting material] × 100

THF represents tetrahydrofuran, GBL represents γ-butyrolactone, Ms represents a methanesulfonyl group ($CH_3SO_2$), and Et represents an ethyl group.

Example 1: Preparation of 1,1-diethoxy-9-methyltridecane (14: $R^1 = R^2$ = Et)

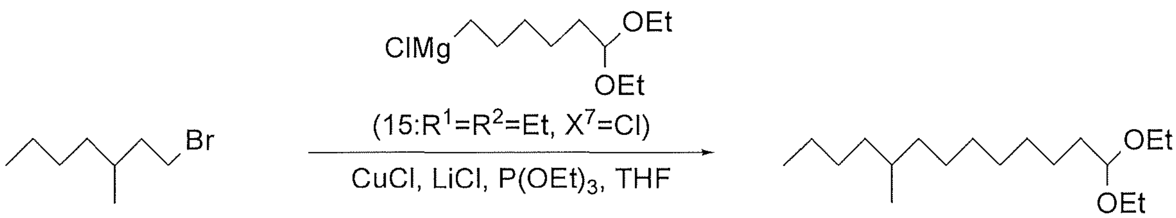


(16) (14:$R^1$=$R^2$=Et)

Cuprous chloride (3.04 g, 0.031 mol), triethyl phosphite ($P(OEt)_3$) (30.46 g, 0.18 mol), lithium chloride (2.11 g, 0.050 mol), tetrahydrofuran (271.60 g), and 1-bromo-3-methylheptane (16: $X^8$ = Br) (492.02 g, 2.47 mol) were placed in the reactor at a room temperature, and a tetrahydrofuran solution (1,447.76 g) of 6,6-diethoxyhexylmagnesium chloride (15:

$R^1 = R^2 = Et$, $X^7 = Cl$) (with 2.72 mol of 6,6-diethoxyhexylmagnesium chloride) was added dropwise at -5 to 10°C. After the completion of the dropwise addition, the reaction mixture was stirred for 1.5 hours at 10 to 20°C. An aqueous solution of acetic acid (prepared from acetic acid (339.50 g) and water (1,018.50 g)) was then added to the reaction mixture, followed by phase separation. The organic layer thus obtained was subjected as such to distillation at a reduced pressure to obtain 1,1-diethoxy-9-methyltridecane (14: $R^1 = R^2 = Et$) (622.23 g, 2.08 mol, purity 95.63%, b.p. = 143.2 to 151.9°C/0.4 kPa (3.0 mmHg)) with a yield of 84.03%.

The following is the spectrum data of 1,1-diethoxy-9-methyltridecane (14: $R^1 = R^2 = Et$) thus prepared.

Nuclear magnetic resonance spectrum: $^1$H-NMR (500 MHz, $CDCl_3$): 0.83 (3H, J = 6.9 Hz), 0.88 (3H, t, J = 6.9 Hz), 1.01-1.13 (2H, m), 1.20 (6H, t, J = 6.9 Hz), 1.18-1.37 (17H, m), 1.60 (2H, dt, J = 9.0 Hz, 6.1 Hz), 3.48 (2H, dq, J = 9.4 Hz, 6.9 Hz), 3.63 (2H, dq, J = 9.4 Hz, 6.9 Hz), 4.50 (1H, t, J = 5.80 Hz); $^{13}$C-NMR (125 MHz, $CDCl_3$): $\delta$ = 14.15, 15.34, 19.69, 23.03, 24.76, 27.03, 29.32, 29.49, 29.60, 29.91, 32.70, 33.58, 36.76, 37.05, 60.77, 102.95.

Mass spectrum: EI-mass spectrum (70 eV): m/z 241 ($M^+$-45), 194, 155, 103, 85, 71, 57, 43, 29.

Infrared absorption spectrum: (D-ATR): νmax = 2954, 2926, 2856, 1485, 1376, 1126, 1063, 1001.

Example 2: Preparation of 9-methyltridecanal (13)

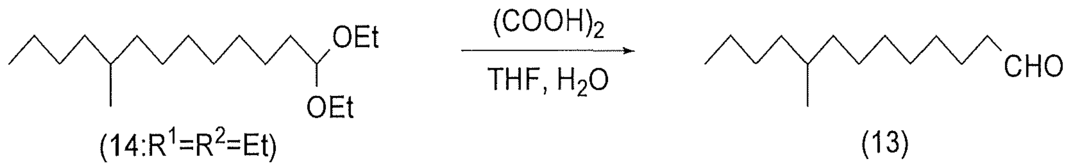


(14:$R^1$=$R^2$=Et) (13)

1,1-Diethoxy-9-methyltridecane (14: $R^1 = R^2 = Et$) (622.23 g, 2.08 mol, purity 95.63%) prepared in Example 1, oxalic acid dihydrate ($(COOH)_2$) (785.54 g, 6.23 mol), tetrahydrofuran (2,077.00 g), and water (2,077.00 g) were placed in a reactor at a room temperature and stirred for 1.5 hours at 60 to 65°C. Hexane (610.85 g) was then added, and the reaction mixture was stirred for 30 minutes. After the completion of the stirring, the reaction mixture was left to stand for phase separation, followed by removal of the aqueous layer to obtain the organic layer. The organic layer thus obtained was concentrated at a reduced pressure, and the resulting concentrate was subjected to distillation at a reduced pressure to obtain 9-methyltridecanal (13) (451.53 g, 2.03 mol, purity 95.51%) with a yield of 97.77%.

The following is the spectrum data of 9-methyltridecanal (13) thus prepared.

Nuclear magnetic resonance spectrum: $^1$H-NMR (500 MHz, $CDCl_3$): $\delta$ = 0.83 (3H, J = 6.5 Hz), 0.88 (3H, t, J = 6.9 Hz), 1.01-1.13 (2H, m), 1.16-1.39 (15H, m), 1.62 (2H, quin-like, J = 7.3 Hz), 2.41 (2H, dt, J = 1.9 Hz, 7.3 Hz), 9.76 (1H, t, J = 1.9 Hz); $^{13}$C-NMR (125 MHz, $CDCl_3$): $\delta$ = 14.14, 19.67, 22.07, 23.02, 26.96, 29.16, 29.31, 29.38, 29.75, 32.68, 36.74, 37.01, 43.90, 202.93.

Mass spectrum: EI-mass spectrum (70 eV): m/z 212 ($M^+$), 197, 184, 155, 137, 124, 109, 95, 81, 69, 55, 43, 29.

Infrared absorption spectrum: (D-ATR): νmax = 2955, 2926, 2856, 2713, 1728, 1465, 1378, 727.

Example 3: Preparation of 10-methyl-2-tetradecanol (11)

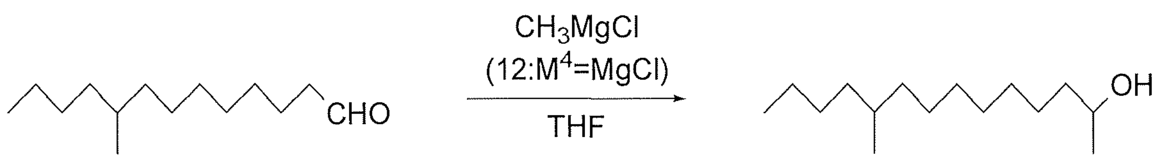


(13) (11)

A tetrahydrofuran solution (765.82 g) of methylmagnesium chloride (12: $M^4$ = MgCl) (with 1.98 mol of methylmagnesium chloride) was placed in a reactor at a room temperature, and 9-methyltridecanal (13) (372.89 g, 1.68 mol, purity 95.51%) prepared in Example 2 was added dropwise at 60 to 65°C. After the completion of the dropwise addition, the reaction mixture was stirred for 2 hours at 60 to 65°C. An aqueous solution of tetrahydrofuran (prepared from tetrahydrofuran (23.63 g) and water (2.36 g)) was then added to the reaction mixture, after which an aqueous solution of acetic acid (prepared from acetic acid (95.92 g) and water (489.62 g)) was added, followed by phase separation. The aqueous layer was removed to obtain the organic layer. The organic layer thus obtained was concentrated at a reduced

pressure, and the resulting concentrate was subjected to distillation at a reduced pressure to obtain 10-methyl-2-tetradecanol (11) (391.02 g, 1.64 mol, purity 95.74%, b.p. = 145.1 to 150.0°C/0.4 kPa (3.0 mmHg)) with a yield of 97.74%.

The following is the spectrum data of 10-methyl-2-tetradecanol (11) thus prepared.

Nuclear magnetic resonance spectrum: $^1$H-NMR (500 MHz, $CDCl_3$): δ = 0.83 (3H, J = 6.5 Hz), 0.88 (3H, t, J = 6.9 Hz), 1.02-1.13 (2H, m), 1.18 (3H, J = 6.5 Hz), 1.20-1.32 (19H, m), 1.56 (1H, br.s), 3.78 (1H, sext-like, J = 6.1 Hz); $^{13}$C-NMR (125 MHz, $CDCl_3$): δ = 14.15, 19.69, 23.03, 23.44, 25.77, 27.04, 29.32, 29.65, 29.93, 32.70, 36.76, 37.05, 39.35, 68.17.

Mass spectrum: EI-mass spectrum (70 eV): m/z 227 ($M^+$-1), 213, 182, 168, 153, 140, 125, 111, 97, 83, 69, 57, 45, 29.

Infrared absorption spectrum: (D-ATR): vmax = 3350, 2958, 2926, 2855, 1465, 1376, 1119, 940, 725.

Example 4: Preparation of 2-chloro-10-methyltetradecane (9: $X^6$ = Cl)

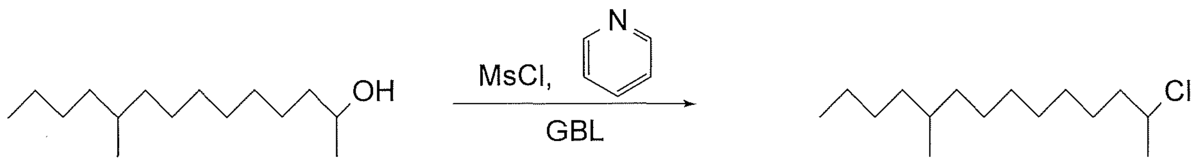


(11) (9:$X^6$=Cl)

10-Methyl-2-tetradecanol (11) (391.02 g, 1.64 mol, purity 95.74%) prepared in Example 3, pyridine (194.47 g, 2.46 mol), and GBL (245.85 g) were placed in a reactor at a room temperature and stirred for 11 minutes at 40°C.

Methanesulfonyl chloride ($CH_3SO_2Cl$) (225.30 g, 1.67 mol) was then added dropwise at 40 to 60°C. After the completion of the dropwise addition, the reaction mixture was heated to 60 to 65°C, and then stirred for 9 hours. After the completion of the stirring, water (409.75 g) and hexane (245.85 g) were added, followed by phase separation. The aqueous layer was removed to obtain the organic layer. The organic layer thus obtained was washed with an aqueous solution of acetic acid (prepared from acetic acid (18.63 g) and water (232.81 g)), and then washed with an aqueous solution of sodium bicarbonate (prepared from sodium bicarbonate (9.31 g) and water (232.81 g)). The organic layer thus obtained was concentrated at a reduced pressure, and the resulting concentrate was subjected to distillation at a reduced pressure to obtain 2-chloro-10-methyltetradecane (9: $X^6$ = Cl) (359.37 g, 1.30 mol, purity 89.52%, b.p. = 118.8 to 120.0°C/0.4 kPa (3.0 mmHg)) with a yield of 79.54%.

The following is the spectrum data of 2-chloro-10-methyltetradecane (9: $X^6$ = Cl) thus prepared.

Nuclear magnetic resonance spectrum: $^1$H-NMR (500 MHz, $CDCl_3$): δ = 0.84 (3H, J = 6.5 Hz), 0.89 (3H, t-like, J = 6.9 Hz), 1.04-1.14 (2H, m), 1.18-1.32 (17H, m), 1.50 (3H, J = 6.5 Hz), 1.63-1.76 (2H, m), 4.02 (1H, tq, J = 6.5 Hz, 6.5 Hz); $^{13}$C-NMR (125 MHz, $CDCl_3$): δ = 14.16, 19.70, 23.04, 25.34, 26.68, 27.03, 29.14, 29.33, 29.54, 29.91, 32.71, 36.77, 37.05, 40.38, 58.93.

Mass spectrum: EI-mass spectrum (70 eV): m/z 231 ($M^+$-15), 210, 188, 153, 139, 111, 97, 83, 69, 55, 41, 29.

Infrared absorption spectrum: (D-ATR): vmax = 2955, 2926, 2856, 1465, 1378, 911, 726, 675, 616.

Example 5: Preparation of 3,11-dimethylpentadecanol (8)

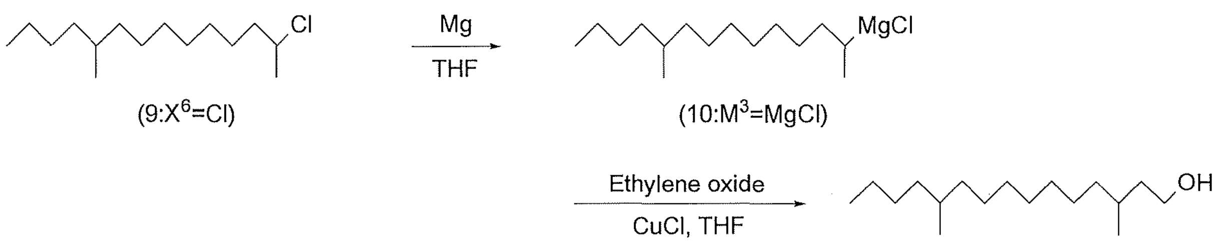


(9:$X^6$=Cl) (10:$M^3$=MgCl)

(8)

Magnesium (25.33 g, 1.04 mol) and tetrahydrofuran (297.84 g) were placed in a reactor at a room temperature and stirred for 14 minutes at 60 to 65°C. After the completion of the stirring, 2-chloro-10-methyltetradecane (9: $X^6$ = Cl) (273.77 g, 0.99 mol, purity 89.52%) prepared in Example 4 was added dropwise to the reactor at 60 to 75°C. After the

completion of the dropwise addition, the reaction mixture was stirred for 2.5 hours at 75 to 80°C to obtain 1,9-dimethyltridecylmagnesium chloride (10: $M^3$ = MgCl).

Cuprous chloride (0.21 g, 0.002 mol) was then added at 0 to 10°C to the reactor, and then ethylene oxide (54.67 g, 1.24 mol) was added dropwise at 0 to 30°C. After the completion of the dropwise addition, the reaction mixture was stirred for 2 hours at 0 to 10°C. After the completion of the stirring, an aqueous solution of acetic acid (prepared from acetic acid (124.10 g) and water (372.30 g)) and hexane (88.25 g) were added to the reaction mixture, followed by phase separation. The aqueous layer was removed to obtain the organic layer. The organic layer thus obtained was concentrated at a reduced pressure, and the resulting concentrate was subjected to distillation at a reduced pressure to obtain 3,11-dimethylpentadecanol (8) (213.50 g, 0.74 mol, purity 88.76%, b.p. = 135.0 to 142.0°C/0.4 kPa (3.0 mmHg)) with a yield of 74.43%.

The following is the spectrum data of 3,11-dimethylpentadecanol (8) thus prepared.

Nuclear magnetic resonance spectrum: $^1$H-NMR (500 MHz, $CDCl_3$): δ = 0.83 (3H, J = 6.9 Hz), 0.85-0.91 (6H, m), 1.03-1.15 (3H, m), 1.16-1.38 (22H, m), 3.62-3.73 (2H, m); $^{13}$C-NMR (125 MHz, $CDCl_3$): δ = 14.15, 19.62, 19.69, 23.03, 26.95, 27.07, 29.32, 29.47, 29.73, 29.94, 30.00, 32.71, 36.76, 37.07, 37.13, 39.96, 61.22.

Mass spectrum: EI-mass spectrum (70 eV): m/z 238 ($M^+$-18), 224, 210, 196, 182, 168, 153, 125, 111, 97, 83, 69, 55, 41.

Infrared absorption spectrum: (D-ATR): νmax = 3334, 2956, 2925, 2855, 1485, 1377, 1058, 724.

Example 6: Preparation of 1-chloro-3,11-dimethylpentadecane (5: $X^3$ = Cl)

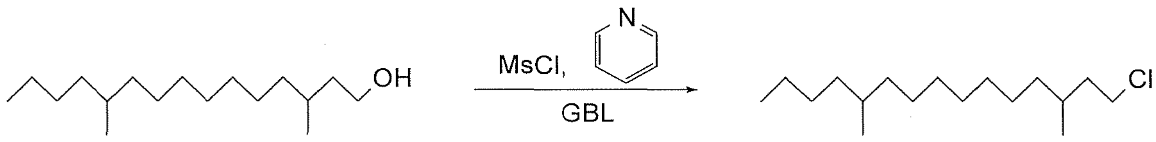


(8) (5:$X^3$=Cl)

3,11-Dimethylpentadecanol (8) (65.00 g, 0.23 mol, purity 88.76%) prepared in Example 5, pyridine (26.70 g, 0.34 mol), and GBL (33.75 g) were placed in a reactor at a room temperature and stirred for 26 minutes at 40°C.

Methanesulfonyl chloride ($CH_3SO_2Cl$) (30.93 g, 0.27 mol) was then added dropwise at 40 to 60°C. After the completion of the dropwise addition, the reaction mixture was heated to 60 to 65°C and stirred for 18 hours. After the completion of the stirring, water (56.25 g) and hexane (33.75 g) were added, followed by phase separation. The aqueous layer was removed to obtain the organic layer. The organic layer thus obtained was washed with an aqueous solution of acetic acid (prepared from acetic acid (2.37 g) and water (29.61 g)), and then washed with an aqueous solution of sodium bicarbonate (prepared from sodium bicarbonate (1.18 g) and water (29.61 g)). The organic layer thus obtained was then concentrated at a reduced pressure, and the resulting concentrate was subjected to distillation at a reduced pressure to obtain 1-chloro-3,11-dimethylpentadecane (5: $X^3$ = Cl) (54.64 g, 0.18 mol, purity 90.06%, b.p. = 125.1 to 138.0°C/0.4 kPa (3.0 mmHg)) with a yield of 79.56%.

The following is the spectrum data of 1-chloro-3,11-dimethylpentadecane (5: $X^3$ = Cl) thus prepared.

Nuclear magnetic resonance spectrum: $^1$H-NMR (500 MHz, $CDCl_3$): δ = 0.84 (3H, J = 6.5 Hz), 0.86-0.91 (6H, m), 1.12-1.17 (3H, m), 1.17-1.40 (19H, m) 1.53-1.62 (1H, m), 1.75-1.83 (1H, m), 3.50-3.62 (1H, m), 4.03 (1H, dt, J = 6.5 Hz, 13.2 Hz); $^{13}$C-NMR (125 MHz, $CDCl_3$): δ = 14.17, 19.08, 19.71, 23.05, 26.83, 27.08, 29.34, 29.70, 29.87, 30.00, 30.37, 32.72, 36.60, 36.78, 37.08, 39.78, 43.37.

Mass spectrum: EI-mass spectrum (70 eV): m/z 274 ($M^+$), 259, 245, 231, 217, 203, 189, 175, 161, 147, 133, 119, 99, 85, 71, 57, 43, 29.

Infrared absorption spectrum: (D-ATR): νmax = 2957, 2925, 2855, 1465, 1378, 1288, 728, 660.

Example 7: Preparation of 1-chloro-2,6,14-trimethyloctadecane (1: $X^1$ = Cl)

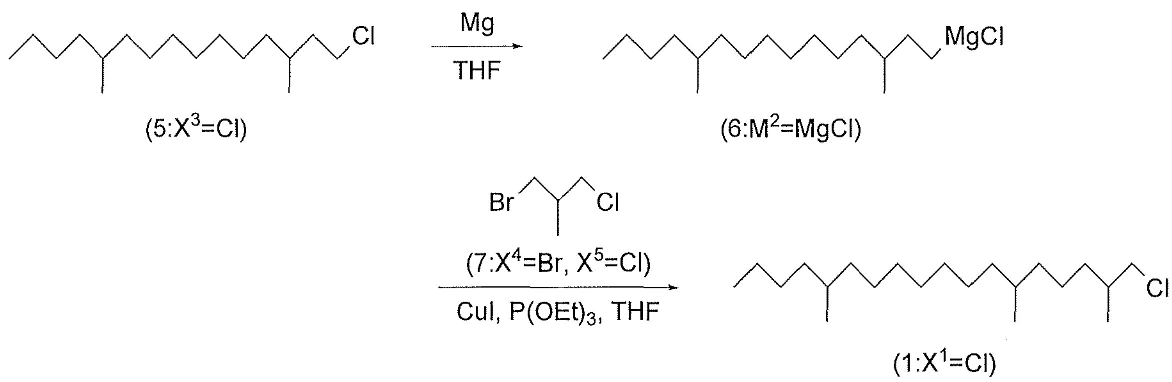


Magnesium (4.22 g, 0.17 gram atoms) and tetrahydrofuran (49.62 g) were placed in a reactor at a room temperature and stirred for 22 minutes at 60 to 65°C. 1-Chloro-3,11-dimethylpentadecane (5: $X^3$ = Cl) (50.49 g, 0.17 mol, purity 90.06%) prepared in Example 6 was then added dropwise at 60 to 75°C to the reactor. After the completion of the dropwise addition, the reaction mixture was stirred for 2 hours at 75 to 80°C to obtain 3,11-dimethylpentadecylmagnesium chloride (6: $M^2$ = MgCl).

Cuprous iodide (0.31 g, 0.0017 mol), triethyl phosphite (0.66 g, 0.0040 mol), tetrahydrofuran (49.62 g), and 1-bromo-3-chloro-2-methylpropane (7: $X^4$ = Br, $X^5$ = Cl) (28.36 g, 0.17 mol) were then placed in another reactor, and then the aforesaid 3,11-dimethylpentadecylmagnesium chloride (6: $M^2$ = MgCl) that was prepared was added dropwise at 5 to 15°C. After the completion of the dropwise addition, the reaction mixture was stirred for 2 hours at 10 to 20°C. An aqueous solution of ammonium chloride (prepared from ammonium chloride (1.65 g) and water (45.57 g)), then 20% by mass hydrochloric acid (1.58 g), and finally an aqueous solution (0.84 g) of 25% by mass sodium hydroxide were then added to the reaction mixture, followed by phase separation. The organic layer thus obtained was concentrated at a reduced pressure, and the resulting concentrate was subjected to distillation at a reduced pressure to obtain 1-chloro-2,6,14-trimethyloctadecane (1: $X^1$ = Cl) (48.56 g, 0.14 mol, purity 92.17%, b.p. = 144.4 to 160.0°C/0.4 kPa (3.0 mmHg)) with a yield of 81.74%.

The following is the spectrum data of 1-chloro-2,6,14-trimethyloctadecane (1: $X^1$ = Cl) thus prepared.

Nuclear magnetic resonance spectrum: $^1$H-NMR (500 MHz, $CDCl_3$): $\delta$ = 0.82-0.86 (6H, m), 0.89 (3H, t-like, J = 6.9 Hz), 1.00 (3H, dd, J = 6.5 Hz, 1.2 Hz), 1.04-1.13 (4H, m), 1.16-1.47 (24H, m), 1.81 (1H, oct-like, J = 6.5 Hz), 3.38-3.43 (1H, m), 3.48 (1H, qd, J = 5.4 Hz, 1.9 Hz); $^{13}$C-NMR (125 MHz, $CDCl_3$): $\delta$ = 14.17, 17.75, 17.82, 19.64, 19.67, 19.71, 23.05, 24.24, 24.26, 27.08, 27.09, 29.34, 29.77, 30.02, 30.04, 32.67, 32.68, 32.73, 34.27, 34.29, 35.53, 36.78, 37.02, 37.08, 37.10, 37.12, 51.28, 51.32.

Mass spectrum: EI-mass spectrum (70 eV): m/z 301 ($M^+$-29) 273, 211, 153, 127, 111, 85, 57, 41.

Infrared absorption spectrum: (D-ATR): νmax = 2956, 2925, 2854, 1463, 1378, 1261, 730, 688.

Example 8: Preparation of 5,13,17-trimethylpentatriacontane (4: *n* = 16)

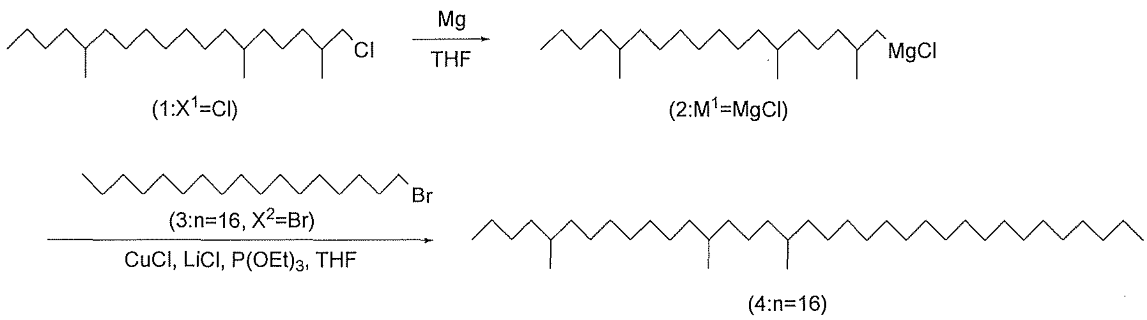


Magnesium (3.31 g, 0.14 gram atoms) and tetrahydrofuran (86.50 g) were placed in a reactor at a room temperature and stirred for 25 minutes at 60 to 65°C. 1-Chloro-2,6,14-trimethyloctadecane (1: $X^1$ = Cl) (46.64 g, 0.13 mol, purity 92.17%) prepared in Example 7 was then added dropwise at 60 to 75°C to the reactor. After the completion of the dropwise addition, the reaction mixture was stirred for 4 hours at 75 to 80°C to obtain 2,6,14-trimethyloctadecylmagnesium chloride (2: $M^1$ = MgCl).

Cuprous chloride (0.15 g, 0.0015 mol), triethyl phosphite (1.46 g, 0.0088 mol), lithium chloride (0.10 g, 0.0024

mol), tetrahydrofuran (100.00 g), and 1-bromoheptadecane (3: *n* = 16, $X^2$ = Br) (41.49 g, 0.13 mol) were then placed in another reactor, and then the aforesaid 2,6,14-trimethyloctadecylmagnesium chloride (2: $M^1$ = MgCl) that was prepared was added dropwise at 15 to 25°C. After the completion of the dropwise addition, the reaction mixture was stirred for 2 hours at 20 to 30°C. An aqueous solution of acetic acid (prepared from acetic acid (1.30 g) and water (35.79 g)), 20% by mass hydrochloric acid (2.72 g), and an aqueous solution (2.72 g) of 25% by mass sodium hydroxide were then added to the reaction mixture, followed by phase separation. The organic layer thus obtained was concentrated at a reduced pressure, and the resulting concentrate was purified with silica gel column chromatography (hexane 100%) to obtain 5,13,17-trimethylpentatriacontane (4: *n* = 16) (63.05 g, 0.11 mol, purity 92.24%) with a yield of 83.68%.

The following is the spectrum data of 5,13,17-trimethylpentatriacontane (4: *n* = 16) thus prepared.

Nuclear magnetic resonance spectrum: $^1$H-NMR (500 MHz, $CDCl_3$): δ = 0.84 (9H, J = 6.9 Hz), 0.86-0.92 (6H, m), 1.01-1.14 (6H, m), 1.16-1.34 (57H, m); $^{13}$C-NMR (125 MHz, $CDCl_3$): δ = 14.12, 14.18, 19.70, 19.72, 19.77, 22.71, 23.06, 24.47, 27.11, 29.35, 29.38, 29.68, 29.72, 29.75, 29.79, 30.06, 31.94, 32.74, 32.76, 32.78, 36.80, 37.08, 37.12, 37.18, 37.39, 37.44.

Mass spectrum: EI-mass spectrum (70 eV): m/z 519 ($M^+$-15), 477, 351, 281, 252, 211, 169, 141, 113, 85, 57, 29.

Infrared absorption spectrum: (D-ATR): ν = 2955, 2923, 2853, 1465, 1379, 721.

## Claims

1. A 1-halo-2,6,14-trimethyloctadecane compound of the following general formula (1):

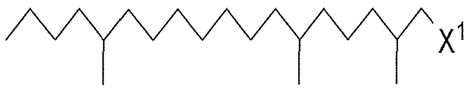
(1)

wherein $X^1$ represents a halogen atom.

2. A process for preparing a 5,13,17-trimethylalkane compound of the following general formula (4):

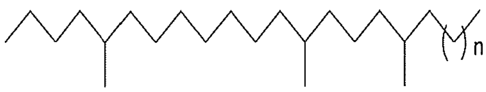
(4)

wherein *n* represents an integer of 14 to 18,
the process comprising
converting a 1-halo-2,6,14-trimethyloctadecane compound of the following general formula (1):

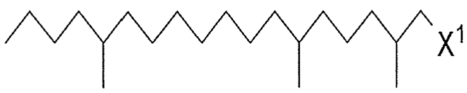
(1)

wherein $X^1$ represents a halogen atom,
into a nucleophilic reagent, 2,6,14-trimethyloctadecyl, of the following general formula (2):

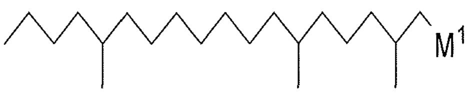
(2)

wherein $M^1$ represents Li or $MgZ^1$, and $Z^1$ represents a halogen atom or a 2,6,14-trimethyloctadecyl group, and subsequently subjecting the nucleophilic reagent, 2,6,14-trimethyloctadecyl compound (2), to a coupling reaction with an electrophilic alkyl reagent of the following general formula (3):

$$CH_3(CH_2)_nX^2 \qquad (3)$$

wherein $X^2$ represents a halogen atom or a *p*-toluenesulfonyloxy group and n is as defined above,
to obtain the aforesaid 5,13,17-trimethylalkane compound (4).

3. A process for preparing a 1-halo-2,6,14-trimethyloctadecane compound of the following general formula (1):

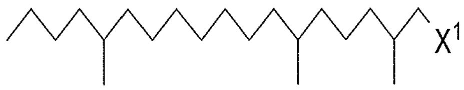 (1)

wherein $X^1$ represents a halogen atom,
the process comprising
converting a 1-halo-3,11-dimethylpentadecane compound of the following general formula (5):

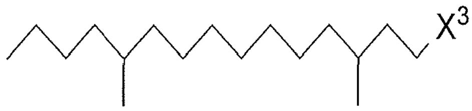 (5)

wherein $X^3$ represents a halogen atom,
into a nucleophilic reagent, 3,11-dimethylpentadecyl, of the following general formula (6):

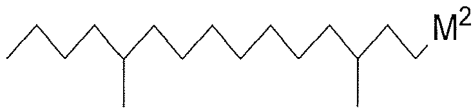 (6)

wherein $M^2$ represents Li or $MgZ^1$, and $Z^1$ represents a halogen atom or a 3,11-dimethylpentadecyl group, and subsequently subjecting the nucleophilic reagent, 3,11-dimethylpentadecyl (6), to a coupling reaction with a 1,3-dihalo-2-methylpropane compound of the following general formula (7):

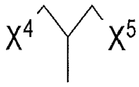 (7)

wherein $X^4$ and $X^5$ represent, independently of each other, a halogen atom,
to obtain the aforesaid 1-halo-2,6,14-trimethyloctadecane compound (1):

**4.** The process according to claim 3 for preparing the 1-halo-2,6,14-trimethyloctadecane compound (1),

the process further comprising
halogenating 3,11-dimethylpentadecanol of the following formula (8):

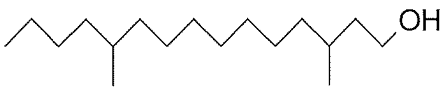 (8)

to obtain the aforesaid 1-halo-3,11-dimethylpentadecane compound (5).

**5.** The process according to claim 4 for preparing the 1-halo-2,6,14-trimethyloctadecane compound (1),

the process further comprising
converting a 2-halo-10-methyltetradecane compound of the following general formula (9):

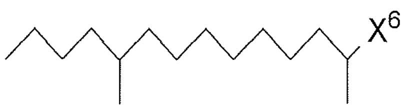 (9)

wherein $X^6$ represents a halogen atom,
into a nucleophilic reagent, 1,9-dimethyltridecyl, of the following general formula (10):

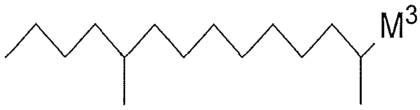

(10)

wherein $M^3$ represents Li or $MgZ^1$, and $Z^1$ represents a halogen atom or a 1,9-dimethyltridecyl group, and subsequently subjecting the nucleophilic reagent, 1,9-dimethyltridecyl (10), to an addition reaction with ethylene oxide to obtain the aforesaid 3,11-dimethylpentadecanol (8).

**6.** The process according to claim 5 for preparing the 1-halo-2,6,14-trimethyloctadecane compound (1),

the process further comprising halogenating 10-methyl-2-tetradecanol of the following formula (11):

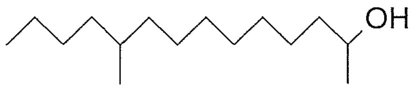

(11)

to obtain the aforesaid 2-halo-10-methyltetradecane compound (9).

**Patentansprüche**

**1.** Eine 1-Halogen-2,6,14-trimethyloctadecanverbindung der nachstehenden allgemeinen Formel (1):

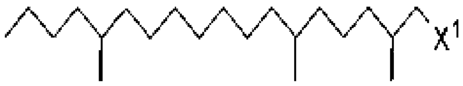

(1)

,

wobei $X^1$ ein Halogenatom darstellt.

**2.** Ein Verfahren zur Herstellung einer 5,13,17-Trimethylalkanverbindung der nachstehenden allgemeinen Formel (4):

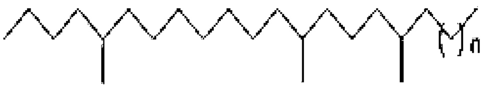

(4)

,

wobei n eine ganze Zahl von 14 bis 18 darstellt, wobei das Verfahren umfasst:

Umwandeln einer 1-Halogen-2,6,14-trimethyloctadenverbindung der nachstehenden allgemeinen Formel (1):

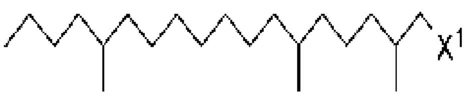

(1)

,

wobei $X^1$ ein Halogenatom darstellt, in ein nukleophiles Reagenz, 2,6,14-Trimethyloctadecyl, der nachstehenden allgemeinen Formel (2):

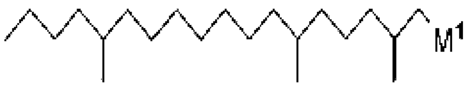

(2)

,

wobei $M^1$ Li oder $MgZ^1$ darstellt und $Z^1$ ein Halogenatom oder eine 2,6,14-Trimethyloctadecylgruppe

darstellt,
und nachfolgend Unterziehen des nukleophilen Reagenzes, 2,6,14-Trimethyloctadecylverbindung (2), einer Kupplungsreaktion mit einem elektrophilen Alkylreagenz der nachstehenden allgemeinen Formel (3):

$$CH_3(CH_2)_nX^2 \qquad (3),$$

wobei $X^2$ ein Halogenatom oder eine p-Toluolsulfonyloxygruppe darstellt und n wie vorstehend definiert ist, um die vorstehend genannte 5,13,17-Trimethylalkanverbindung (4) zu erhalten.

**3.** Ein Verfahren zur Herstellung einer 1-Halogen-2,6,14-trimethyloctadecanverbindung der nachstehenden allgemeinen Formel (1):

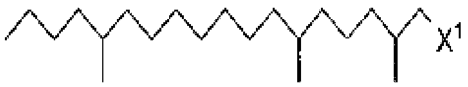
(1)

,

wobei $X^1$ ein Halogenatom darstellt,
wobei das Verfahren umfasst:

Umwandeln einer 1-Halogen-3,11-dimethylpentadecanverbindung der nachstehenden allgemeinen Formel (5):

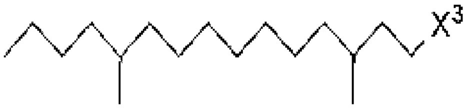
(5)

,

wobei $X^3$ ein Halogenatom darstellt,
in ein nukleophiles Reagenz, 3,11-Dimethylpentadecyl, der nachstehenden allgemeinen Formel (6):

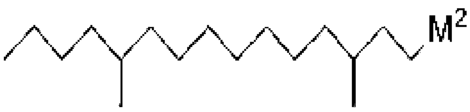
(6)

,

wobei $M^2$ Li oder $MgZ^1$ darstellt und $Z^1$ ein Halogenatom oder eine 3,11-Dimethylpentadecylgruppe darstellt, und nachfolgend Unterziehen des nukleophilen Reagenzes, 3,11-Dimethylpentadecyl (6), einer Kupplungsreaktion mit einer 1,3-Dihalogen-2-methylpropanverbindung der nachstehenden allgemeinen Formel (7):

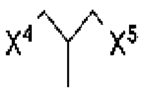
(7)

,

wobei $X^4$ und $X^5$ unabhängig voneinander ein Halogenatom darstellen,
um die vorstehend genannte 1-Halogen-2,6,14-trimethyloctadecanverbindung (1) zu erhalten.

**4.** Das Verfahren gemäß Anspruch 3 zur Herstellung der 1-Halogen-2,6,14-trimethyloctadecanverbindung (1), wobei das Verfahren ferner umfasst:

Halogenieren von 3,11-Dimethylpentadecanol der nachstehenden Formel (8):

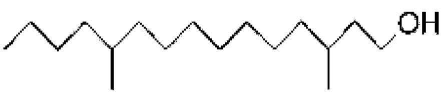
(8)

,

um die vorstehend genannte 1-Halogen-3,11-dimethylpentadecanverbindung (5) zu erhalten.

**5.** Das Verfahren gemäß Anspruch 4 zur Herstellung der 1-Halogen-2,6,14-trimethyloctadecanverbindung (1), wobei das Verfahren ferner umfasst:

Umwandeln einer 2-Halogen-10-methyltetradecanverbindung der nachstehenden allgemeinen Formel (9):

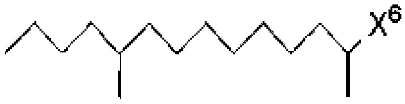

(9)

,

wobei $X^6$ ein Halogenatom darstellt,
in ein nukleophiles Reagenz, 1,9-Dimethyltridecyl, der nachstehenden allgemeinen Formel (10):

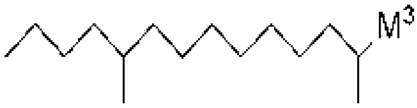

(10)

,

wobei $M^3$ Li oder $MgZ^1$ darstellt und $Z^1$ ein Halogenatom oder eine 1,9-Dimethyltridecylgruppe darstellt, und nachfolgend Unterziehen des nukleophilen Reagenzes, 1,9-Dimethyltridecyl (10), einer Additionsreaktion mit Ethylenoxid, um das vorstehend genannte 3,11-Dimethylpentadecanol (8) zu erhalten.

**6.** Das Verfahren gemäß Anspruch 5 zur Herstellung der 1-Halogen-2,6,14-Trimethyloctadecanverbindung (1), wobei das Verfahren ferner umfasst:

Halogenieren von 10-Methyl-2-tetradecanol der nachstehenden Formel (11):

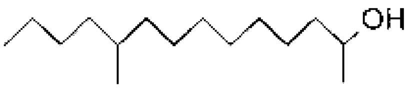

**(11)**

,

um die vorstehend genannte 2-Halogen-10-methyltetradecanverbindung (9) zu erhalten.

## Revendications

**1.** Composé de 1-halo-2,6,14-triméthyloctadécane de formule générale (1) suivante :

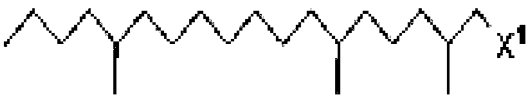

(1)

dans lequel $X^1$ représente un atome d'halogène.

**2.** Procédé de préparation d'un composé de 5,13,17-triméthylalcane de formule générale (4) suivante :

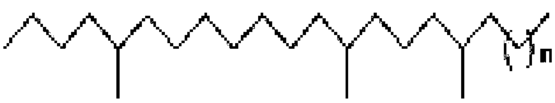

(4)

dans lequel n représente un nombre entier de 14 à 18,
le procédé comprenant
la conversion d'un composé de 1-halo-2,6,14-triméthyloctadécane de formule générale (1) suivante :

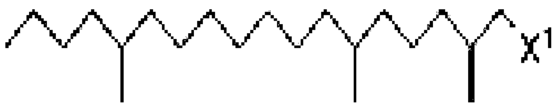 (1)

dans lequel $X^1$ représente un atome d'halogène,
en un réactif nucléophile, le 2,6,14-triméthyloctadécyle, de formule générale (2) suivante :

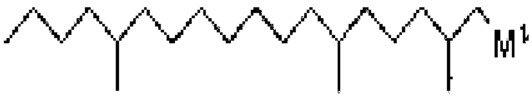 (2)

dans lequel $M^1$ représente Li ou $MgZ^1$, et $Z^1$ représente un atome d'halogène ou un groupe 2,6,14-triméthyloctadécyle, puis la soumission du réactif nucléophile, le composé (2) de 2,6,14-triméthyloctadécyle, à une réaction de couplage avec un réactif alkyle électrophile de formule générale (3) suivante :

$CH_3(CH_2)_nX^2$ (3)

dans lequel $X^2$ représente un atome d'halogène ou un groupe p-toluènesulfonyloxy, et n est défini comme ci-dessus,
pour obtenir le composé (4) de 5,13,17-triméthylalcane susmentionné.

3. Procédé de préparation d'un composé de 1-halo-2,6,14-triméthyloctadécane de formule générale (1) suivante :

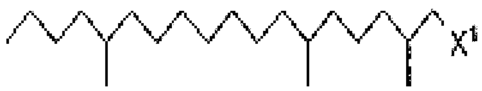 (1)

dans lequel $X^1$ représente un atome d'halogène,
le procédé comprenant
la conversion d'un composé de 1-halo-3,11-diméthylpentadécane de formule générale (5) suivante :

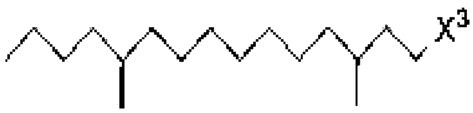 (5)

dans lequel $X^3$ représente un atome d'halogène,
en un réactif nucléophile, le 3,11-diméthylpentadécyle, de formule générale (6) suivante :

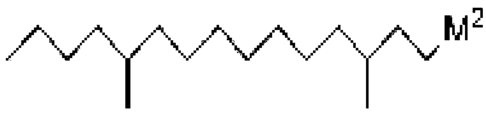 (6)

dans lequel $M^2$ représente Li ou $MgZ^1$, et $Z^1$ représente un atome d'halogène ou un groupe 3,11-diméthylpentadécyle, puis la soumission du réactif nucléophile, le 3,11-diméthylpentadécyle (6), à une réaction de couplage avec un composé de 1,3-dihalo-2-méthylpropane de formule générale (7) suivante :

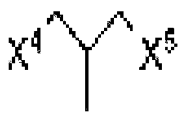 (7)

dans lequel $X^4$ et $X^5$ représente, indépendamment l'un de l'autre, un atome d'halogène, pour obtenir le composé (1) de 1-halo-2,6,14-triméthyloctadécane susmentionné.

4. Procédé selon la revendication 3 pour la préparation du composé (1) de 1-halo-2,6,14-triméthyloctadécane,

le procédé comprenant en outre
l'halogénation du 3,11-diméthylpentadécanol de formule (8) suivante :

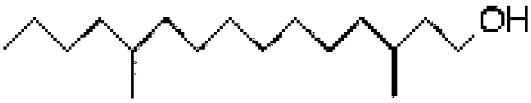

(8)

pour obtenir le composé (5) de 1-halo-3,11-diméthylpentadécane susmentionné.

**5.** Procédé selon la revendication 4 pour la préparation du composé (1) de 1-halo-2,6,14-triméthyloctadécane,

le procédé comprenant en outre
la conversion d'un composé de 2-halo-10-méthyltétradécane de formule générale (9) suivante :

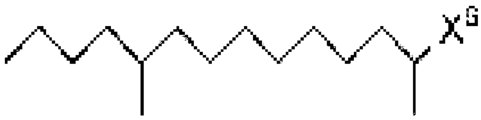

(9)

dans lequel $X^6$ représente un atome d'halogène,
en un réactif nucléophile, le 1,9-diméthyltridécyle, de formule générale (10) suivante :

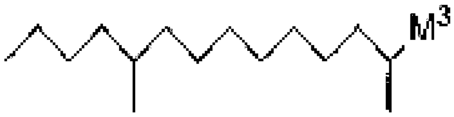

(10)

dans lequel $M^3$ représente Li ou $MgZ^1$, et $Z^1$ représente un atome d'halogène ou un groupe 1,9-diméthyltridécyle, puis la soumission du réactif nucléophile, le 1,9-diméthyltridécyle (10), à une réaction d'addition avec de l'oxyde d'éthylène pour obtenir le 3,11-diméthylpentadécanol (8) susmentionné.

**6.** Procédé selon la revendication 5 pour la préparation du composé (1) de 1-halo-2,6,14-triméthyloctadécane,

le procédé comprenant en outre
l'halogénation du 10-méthyl-2-tétradécanol de formule (11) suivante :

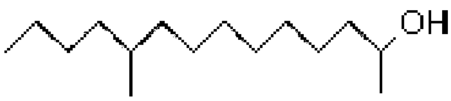

(11)

pour obtenir le composé (9) de 2-halo-10-méthyltétradécane susmentionné.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NEIL D TSUTSUI et al.** *BMC Biology*, 2009, vol. 7, 71
- **NEIL D TSUTSUI et al.** *J. Chem. Ecol.*, 2010, vol. 36, 751-758
- **E. SUNAMURA et al.** *Insectes Sociaux*, 2009, vol. 56, 143-147
- **DENNIS H. BURNS et al.** *J. Am. Chem. Soc.*, 1997, vol. 119, 2125-2133